# EUROPEAN PATENT SPECIFICATION

(11) **EP 1 648 492 B1**
(45) Date of publication and mention of the grant of the patent: **11.12.2013**
(21) Application number: 04777402.1
(22) Date of filing: 01.07.2004
(51) Int. Cl.: A61K 38/08, A61P 35/00

(54) **SYNTHETIC ANTIPROLIFERATIVE FACTOR GLYCOPEPTIDES AND METHODS OF USE**
SYNTHETISCHE ANTIPROLIFERATIVER FAKTOR GLYKOPEPTIDEN UND ANWENDUNGSVERFAHREN
GLYCOPEPTIDES SYNTHETIQUES DU FACTEUR ANTIPROLIFERATIF ET LEURS METHODES D'UTILISATION

(30) Priority: 01.07.2003 US 484010 P; 30.10.2003 US 515850 P; 08.05.2004 US 569363 P
(43) Date of publication of application: 26.04.2006
(73) Proprietor: University of Maryland, Baltimore, Baltimore, MD 21201-1691 (US); The United States of America, as represented by The Secretary, Department of Health and Human Services, Rockville, MD 20852-3804 (US); The United States of America, as represented by the Department of Veterans Affairs, Washington, D.C. 20420 (US)
(72) Inventor: KEAY, Susan, K., Ellicott City, MD 21042 (US); SZEKELY, Zoltan, Gaithersburg, MD 20879 (US); MICHEJDA, Christopher, North Potomac, MD 20878 (US); CONRADS, Thomas P., Reston VA 20191 (US); VEENSTRA, Timothy D., Jefferson Maryland 21755 (US)
(74) Representative: Woods, Geoffrey Corlett
(86) International application number: PCT/US2004/021239
(87) International publication number: WO 2005/016248

(56) References cited:
- US-A- 5 962 645
- US-A1- 2002 016 443
- SAITOH T ET AL: "Molecular cloning and characterization of human Frizzled-8 gene on chromosome 10p11.2" INTERNATIONAL JOURNAL OF ONCOLOGY, EDITORIAL ACADEMY OF THE INTERNATIONAL JOURNAL OF ONCOLOGY,, GR, vol. 18, no. 5, May 2001 (2001-05), pages 991-996, XP009096208 ISSN: 1019-6439
- KEAY SUSAN K ET AL: "An antiproliferative factor from interstitial cystitis patients is a frizzled 8 protein-related sialoglycopeptide." PROCEEDINGS OF THE NATIONAL ACADEMY OF SCIENCES OF THE UNITED STATES OF AMERICA 10 AUG 2004, vol. 101, no. 32, 10 August 2004 (2004-08-10), pages 11803-11808, XP002470028 ISSN: 0027-8424
- KEAY S. ET AL: 'Bladder epithelial cells from patients with interstitial cystitis produce an inhibitor of heparin-binding epidermal growth factor-like growth factor production' J UROL. vol. 164, no. 6, December 2000, pages 2112 - 2118, XP002988958

### SEQUENCE LISTING

<110> Keay, Susan Michejda, Christopher Szekely, Zoltan
<120> A NOVEL ANTIPROLIFERATIVE FACTOR AND METHODS OF USE
<130> HO-P03002WO0
<140> Not Assigned
   <141> 2004-07-01
<150> US 60/484,010
   <151> 2003-07-01
<150> US 60/515,850
   <151> 2003-10-29
<150> US 60/569,363
   <151> 2004-05-07
<160> 9
<170> PatentIn version 3.1
<210> 1
   <211> 9
   <212> PRT
   <213> ARTIFICIAL SEQUENCE
<220>
   <223> Peptide
<400> 1
   Thr Val Pro Ala Ala Val Val Val Ala
   1               5
<210> 2
   <211> 27
   <212> DNA
   <213> ARTIFICIAL SEQUENCE
<220>
   <223> Oligonucleotide
<400> 2
   accgtgcccg ccgcggtggt ggtcgcc 27
<210> 3
   <211> 9
   <212> PRT
   <213> ARTIFICIAL SEQUENCE
<220>
   <223> Peptide
<400> 3
   Ser Val Pro Ala Ala Val Val Val Ala
   1               5
<210> 4
   <211> 9
   <212> PRT
   <213> ARTIFICIAL SEQUENCE
<220>
   <223> Peptide
<400> 4
   Thr Val Pro Ala Ala Val Val Leu Ala
   1               5
<210> 5
   <211> 9
   <212> PRT
   <213> ARTIFICIAL SEQUENCE
<220>
   <223> Peptide .
<400> 5
   Ser Leu Pro Ala Ala Val Val Val Ala
   1               5
<210> 6
   <211> 10
   <212> PRT
   <213> ARTIFICIAL SEQUENCE
<220>
   <223> Peptide
<400> 6
   Cys Thr Val Pro Ala Ala Val Val Val Ala
   1               5                   10
<210> 7
   <211> 694
   <212> PRT
   <213> HUMAN
<400> 7
   Met Glu Trp Gly Tyr Leu Leu Glu Val Thr Ser Leu Leu Ala Ala Leu
   1               5                   10                  15
   Ala Leu Leu Gln Arg Ser Ser Gly Ala Ala Ala Ala Ser Ala Lys Glu
               20                  25                  30
   Leu Ala Cys Gln Glu Ile Thr Val Pro Leu Cys Lys Gly Ile Gly Tyr
           35                  40                  45
   Asn Tyr Thr Tyr Met Pro Asn Gln Phe Asn His Asp Thr Gln Asp Glu
       50                  55                  60
   Ala Gly Leu Glu Val His Gln Phe Trp Pro Leu Val Glu Ile Gln Cys
   65                  70                  75                  80
   Ser Pro Asp Leu Lys Phe Phe Leu Cys Ser Met Tyr Thr Pro Ile Cys
                   85                  90                  95
   Leu Glu Asp Tyr Lys Lys Pro Leu Pro Pro Cys Arg Ser Val Cys Glu
               100                 105                 110
   Arg Ala Lys Ala Gly Cys Ala Pro Leu Met Arg Gln Tyr Gly Phe Ala
           115                 120                 125
   Trp Pro Asp Arg Met Arg Cys Asp Arg Leu Pro Glu Gln Gly Asn Pro
       130                 135                 140
   Asp Thr Leu Cys Met Asp Tyr Asn Arg Thr Asp Leu Thr Thr Ala Ala
   145                 150                 155                 160
   Pro Ser Pro Pro Arg Arg Leu Pro Pro Pro Pro Pro Gly Glu Gln Pro
                   165                 170                 175
   Pro Ser Gly Ser Gly His Gly Arg Pro Pro Gly Ala Arg Pro Pro His
               180                 185                 190
   Arg Gly Gly Gly Arg Gly Gly Gly Gly Gly Asp Ala Ala Ala Pro Pro
           195                 200                 205
   Ala Arg Gly Gly Gly Gly Gly Gly Lys Ala Arg Pro Pro Gly Gly Gly
       210                 215                 220
   Ala Ala Pro Cys Glu Pro Gly Cys Gln Cys Arg Ala Pro Met Val Ser
   225                 230                 235                 240
   Val Ser Ser Glu Arg His Pro Leu Tyr Asn Arg Val Lys Thr Gly Gln
                   245                 250                 255
   Ile Ala Asn Cys Ala Leu Pro Cys His Asn Pro Phe Phe Ser Gln Asp
               260                 265                 270
   Glu Arg Ala Phe Thr Val Phe Trp Ile Gly Leu Trp Ser Val Leu Cys
           275                 280                 285
   Phe Val Ser Thr Phe Ala Thr Val Ser Thr Phe Leu Ile Asp Met Glu
       290                 295                 300
   Arg Phe Lys Tyr Pro Glu Arg Pro Ile Ile Phe Leu Ser Ala Cys Tyr
   305                 310                 315                 320
   Leu Phe Val Ser Val Gly Tyr Leu Val Arg Leu Val Ala Gly His Glu
                   325                 330                 335
   Lys Val Ala Cys Ser Gly Gly Ala Pro Gly Ala Gly Gly Ala Gly Gly
               340                 345                 350
   Ala Gly Gly Ala Ala Ala Gly Ala Gly Ala Ala Gly Ala Gly Ala Gly
           355                 360                 365
   Gly Pro Gly Gly Arg Gly Glu Tyr Glu Glu Leu Gly Ala Val Glu Gln
       370                 375                 380
   His Val Arg Tyr Glu Thr Thr Gly Pro Ala Leu Cys Thr Val Val Phe
   385                 390                 395                 400
   Leu Leu Val Tyr Phe Phe Gly Met Ala Ser Ser Ile Trp Trp Val Ile
                   405                 410                 415
   Leu Ser Leu Thr Trp Phe Leu Ala Ala Gly Met Lys Trp Gly Asn Glu
               420                 425                 430
   Ala Ile Ala Gly Tyr Ser Gln Tyr Phe His Leu Ala Ala Trp Leu Val
           435                 440                 445
   Pro Ser Val Lys Ser Ile Ala Val Leu Ala Leu Ser Ser Val Asp Gly
       450                 455                 460
   Asp Pro Val Ala Gly Ile Cys Tyr Val Gly Asn Gln Ser Leu Asp Asn
   465                 470                 475                 480
   Leu Arg Gly Phe Val Leu Ala Pro Leu Val Ile Tyr Leu Phe Ile Gly
                   485                 490                 495
   Thr Met Phe Leu Leu Ala Gly Phe Val Ser Leu Phe Arg Ile Arg Ser
               500                 505                 510
   Val Ile Lys Gln Gln Asp Gly Pro Thr Lys Thr His Lys Leu Glu Lys
   515 520 525
   Leu Met Ile Arg Leu Gly Leu Phe Thr Val Leu Tyr Thr Val Pro Ala
   530 535 540
   Ala Val Val Val Ala Cys Leu Phe Tyr Glu Gln His Asn Arg Pro Arg
   545 550 555 560
   Trp Glu Ala Thr His Asn Cys Pro Cys Leu Arg Asp Leu Gln Pro Asp
   565 570 575
   Gln Ala Arg Arg Pro Asp Tyr Ala Val Phe Met Leu Lys Tyr Phe Met
   580 585 590
   Cys Leu Val Val Gly Ile Thr Ser Gly Val Trp Val Trp Ser Gly Lys
   595 600 605
   Thr Leu Glu Ser Trp Arg Ser Leu Cys Thr Arg Cys Cys Trp Ala Ser
   610 615 620
   Lys Gly Ala Ala Val Gly Gly Gly Ala Gly Ala Thr Ala Ala Gly Gly
   625 630 635 640
   Gly Gly Gly Pro Gly Gly Gly Gly Gly Gly Gly Pro Gly Gly Gly Gly
   645 650 655
   Gly Pro Gly Gly Gly Gly Gly Ser Leu Tyr Ser Asp Val Ser Thr Gly
   660 665 670
   Leu Thr Trp Arg Ser Gly Thr Ala Ser Ser Val Ser Tyr Pro Lys Gln
   675 680 685
   Met Pro Leu Ser Gln Val
   690
<210> 8
   <211> 3195
   <212> DNA
   <213> HUMAN
<400> 8
   acagcatgga gtggggttac ctgttggaag tgacctcgct gctggccgcc ttggcgctgc 60
   tgcagcgctc tagcggcgct gcggccgcct cggccaagga gctggcatgc caagagatca 120
   ccgtgccgct gtgtaagggc atcggctaca actacaccta catgcccaat cagttcaacc 180
   acgacacgca agacgaggcg ggcctggagg tgcaccagtt ctggccgctg gtggagatcc 240
   agtgctcgcc cgatctcaag ttcttcctgt gcagcatgta cacgcccatc tgcctagagg 300
   actacaagaa gccgctgccg ccctgccgct cggtgtgcga gcgcgccaag gccggctgcg 360
   cgccgctcat gcgccagtac ggcttcgcct ggcccgaccg catgcgctgc gaccggctgc 420
   ccgagcaagg caaccctgac acgctgtgca tggactacaa ccgcaccgac ctaaccaccg 480
   ccgcgcccag cccgccgcgc cgcctgccgc cgccgccgcc cggcgagcag ccgccttcgg 540
   gcagcggcca cggccgcccg ccgggggcca ggcccccgca ccgcggaggc ggcaggggcg 600
   gtggcggcgg ggacgcggcg gcgcccccag ctcgcggcgg cggcggtggc gggaaggcgc 660
   ggccccctgg cggcggcgcg gctccctgcg agcccgggtg ccagtgccgc gcgcctatgg 720
   tgagcgtgtc cagcgagcgc cacccgctct acaaccgcgt caagacaggc cagatcgcta 780
   actgcgcgct gccctgccac aacccctttt tcagccagga cgagcgcgcc ttcaccgtct 840
   tctggatcgg cctgtggtcg gtgctctgct tcgtgtccac cttcgccacc gtctccacct 900
   tccttatcga catggagcgc ttcaagtacc cggagcggcc cattatcttc ctctcggcct 960
   gctacctctt cgtgtcggtg ggctacctag tgcgcctggt ggcgggccac gagaaggtgg 1020
   cgtgcagcgg tggcgcgccg ggcgcggggg gcgctggggg cgcgggcggc gcggcggcgg 1080
   gcgcgggcgc ggcgggcgcg ggcgcgggcg gcccgggcgg gcgcggcgag tacgaggagc 1140
   tgggcgcggt ggagcagcac gtgcgctacg agaccaccgg ccccgcgctg tgcaccgtgg 1200
   tcttcttgct ggtctacttc ttcggcatgg ccagctccat ctggtgggtg atcttgtcgc 1260
   tcacatggtt cctggcggcc ggtatgaagt ggggcaacga agccatcgcc ggctactcgc 1320
   agtacttcca cctggccgcg tggcttgtgc ccagcgtcaa gtccatcgcg gtgctggcgc 1380
   tcagctcggt ggacggcgac ccggtggcgg gcatctgcta cgtgggcaac cagagcctgg 1440
   acaacctgcg cggcttcgtg ctggcgccgc tggtcatcta cctcttcatc ggcaccatgt 1500
   tcctgctggc cggcttcgtg tccctgttcc gcatccgctc ggtcatcaag caacaggacg 1560
   gccccaccaa gacgcacaag ctggagaagc tgatgatccg cctgggcctg ttcaccgtgc 1620
   tctacaccgt gcccgccgcg gtggtggtcg cctgcctctt ctacgagcag cacaaccgcc 1680
   cgcgctggga ggccacgcac aactgcccgt gcctgcggga cctgcagccc gaccaggcac 1740
   gcaggcccga ctacgccgtc ttcatgctca agtacttcat gtgcctagtg gtgggcatca 1800
   cctcgggcgt gtgggtctgg tccggcaaga cgctggagtc ctggcgctcc ctgtgcaccc 1860
   gctgctgctg ggccagcaag ggcgccgcgg tgggcggggg cgcgggcgcc acggccgcgg 1920
   ggggtggcgg cgggccgggg ggcggcggcg gcgggggacc cggcggcggc ggggggccgg 1980
   gcggcggcgg gggctccctc tacagcgacg tcagcactgg cctgacgtgg cggtcgggca 2040
   cggcgagctc cgtgtcttat ccaaagcaga tgccattgtc ccaggtctga gcggagggga 2100
   gggggcgccc aggaggggtg gggagggggg cgaggagacc caagtgcagc gaagggacac 2160
   ttgatgggct gaggttccca ccccttcaca gtgttgattg ctattagcat gataatgaac 2220
   tcttaatggt atccattagc tgggacttaa atgactcact tagaacaaag tacctggcat 2280
   tgaagcctcc cagacccagc cccttttcct ccattgatgt gcggggagct cctcccgcca 2340
   cgcgttaatt tctgttggct gaggagggtg gactctgcgg cgtttccaga acccgagatt 2400
   tggagccctc cctggctgca cttggctggg tttgcagtca gatacacaga tttcacctgg 2460
   gagaacctct ttttctccct cgactcttcc tacgtaaact cccacccctg acttaccctg 2520
   gaggaggggt gaccgccacc tgatgggatt gcacggtttg ggtattctta atgaccaggc 2580
   aaatgcctta agtaaacaaa caagaaatgt cttaattata caccccacgt aaatacgggt 2640
   ttcttacatt agaggatgta tttatataat tatttgttaa attgtaaaaa aaaaaagtgt 2700
   aaaatatgta tatatccaaa gatatagtgt gtacattttt ttgtaaaaag tttagaggct 2760
   tacccctgta agaacagata taagtattct attttgtcaa taaaatgact tttgataaat 2820
   gatttaacca ttgccctctc ccccgcctct tctgagctgt cacctttaaa gtgcttgcta 2880
   aggacgcatg gggaaaatgg acattttctg gcttgtcatt ctgtacactg accttaggca 2940
   tggagaaaat tacttgttaa actctagttc ttaagttgtt agccaagtaa atatcattgt 3000
   tgaactgaaa tcaaaattga gtttttgcac cttccccaaa gacggtgttt ttcatgggag 3060
   ctcttttctg atccatggat aacaactctc actttagtgg atgtaaatgg aacttctgca 3120
   aggcagtaat tccccttagg ccttgttatt tatcctgcat ggtatcacta aaggtttcaa 3180
   aaccctgaaa aaaaa 3195
<210> 9
   <211> 685
   <212> PRT
   <213> MOUSE
<400> 9
   Met Glu Trp Gly Tyr Leu Leu Glu Val Thr Ser Leu Leu Ala Ala Leu
   1 5 10 15
   Ala Val Leu Gln Arg Ser Ser Gly Ala Ala Ala Ala Ser Ala Lys Glu
   20 25 30
   Leu Ala Cys Gln Glu Ile Thr Val Pro Leu Cys Lys Gly Ile Gly Tyr
   35 40 45
   Asn Tyr Thr Tyr Met Pro Asn Gln Phe Asn His Asp Thr Gln Asp Glu
   50 55 60
   Ala Gly Leu Glu Val His Gln Phe Trp Pro Leu Val Glu Ile Gln Cys
   65                  70                  75                  80
   Ser Pro Asp Leu Lys Phe Phe Leu Cys Ser Met Tyr Thr Pro Ile Cys
                   85                  90                  95
   Leu Glu Asp Tyr Lys Lys Pro Leu Pro Pro Cys Arg Ser Val Cys Glu
               100                 105                 110
   Arg Ala Lys Ala Gly Cys Ala Pro Leu Met Arg Gln Tyr Gly Phe Ala
           115                 120                 125
   Trp Pro Asp Arg Met Arg Cys Asp Arg Leu Pro Glu Gln Gly Asn Pro
       130                 135                 140
   Asp Thr Leu Cys Met Asp Tyr Asn Arg Thr Asp Leu Thr Thr Ala Ala
   145                 150                 155                 160
   Pro Ser Pro Pro Arg Arg Leu Pro Pro Pro Pro Pro Pro Gly Glu Gln
                   165                 170                 175
   Pro Pro Ser Gly Ser Gly His Ser Arg Pro Pro Gly Ala Arg Pro Pro
               180                 185                 190
   His Arg Gly Gly Ser Ser Arg Gly Ser Gly Asp Ala Ala Ala Ala Pro
           195                 200                 205
   Pro Ser Arg Gly Gly Lys Ala Arg Pro Pro Gly Gly Gly Ala Ala Pro
       210                 215                 220
   Cys Glu Pro Gly Cys Gln Cys Arg Ala Pro Met Val Ser Val Ser Ser
   225                 230                 235                 240
   Glu Arg His Pro Leu Tyr Asn Arg Val Lys Thr Gly Gln Ile Ala Asn
                   245                 250                 255
   Cys Ala Leu Pro Cys His Asn Pro Phe Phe Ser Gln Asp Glu Arg Ala
               260                 265                 270
   Phe Thr Val Phe Trp Ile Gly Leu Trp Ser Val Leu Cys Phe Val Ser
           275                 280                 285
   Thr Phe Ala Thr Val Ser Thr Phe Leu Ile Asp Met Glu Arg Phe Lys
       290                 295                 300
   Tyr Pro Glu Arg Pro Ile Ile Phe Leu Ser Ala Cys Tyr Leu Phe Val
   305                 310                 315                 320
   Ser Val Gly Tyr Leu Val Arg Leu Val Ala Gly His Glu Lys Val Ala
                   325                 330                 335
   Cys Ser Gly Gly Ala Pro Gly Ala Gly Gly Arg Gly Gly Ala Gly Gly
               340                 345                 350
   Ala Ala Ala Ala Gly Ala Gly Ala Ala Gly Arg Gly Ala Ser Ser Pro
           355                 360                 365
   Gly Ala Arg Gly Glu Tyr Glu Glu Leu Gly Ala Val Glu Gln His Val
       370                 375                 380
   Arg Tyr Glu Thr Thr Gly Pro Ala Leu Cys Thr Val Val Phe Leu Leu
   385                 390                 395                 400
   Val Tyr Phe Phe Gly Met Ala Ser Ser Ile Trp Trp Val Ile Leu Ser
                   405                 410                 415
   Leu Thr Trp Phe Leu Ala Ala Gly Met Lys Trp Gly Asn Glu Ala Ile
               420                 425                 430
   Ala Gly Tyr Ser Gln Tyr Phe His Leu Ala Ala Trp Leu Val Pro Ser
           435                 440                 445
   Val Lys Ser Ile Ala Val Leu Ala Leu Ser Ser Val Asp Gly Asp Pro
       450                 455                 460
   Val Ala Gly Ile Cys Tyr Val Gly Asn Gln Ser Leu Asp Asn Leu Arg
   465                 470                 475                 480
   Gly Phe Val Leu Ala Pro Leu Val Ile Tyr Leu Phe Ile Gly Thr Met
                   485                 490                 495
   Phe Leu Leu Ala Gly Phe Val Ser Leu Phe Arg Ile Arg Ser Val Ile
               500                 505                 510
   Lys Gln Gln Gly Gly Pro Thr Lys Thr His Lys Leu Glu Lys Leu Met
           515                 520                 525
   Ile Arg Leu Gly Leu Phe Thr Val Leu Tyr Thr Val Pro Ala Ala Val
       530                 535                 540
   Val Val Ala Cys Leu Phe Tyr Glu Gln His Asn Arg Pro Arg Trp Glu
   545                 550                 555                 560
   Ala Thr His Asn Cys Pro Cys Leu Arg Asp Leu Gln Pro Asp Gln Ala
                   565                 570                 575
   Arg Arg Pro Asp Tyr Ala Val Phe Met Leu Lys Tyr Phe Met Cys Leu
               580                 585                 590
   Val Val Gly Ile Thr Ser Gly Val Trp Val Trp Ser Gly Lys Thr Leu
           595                 600                 605
   Glu Ser Trp Arg Ala Leu Cys Thr Arg Cys Cys Trp Ala Ser Lys Gly
       610                 615                 620
   Ala Ala Val Gly Ala Gly Ala Gly Gly Ser Gly Pro Gly Gly Ser Gly
   625                 630                 635                 640
   Pro Gly Pro Gly Gly Gly Gly Gly His Gly Gly Gly Gly Gly Ser Leu
                   645                 650                 655
   Tyr Ser Asp Val Ser Thr Gly Leu Thr Trp Arg Ser Gly Thr Ala Ser
               660                 665                 670
   Ser Val Ser Tyr Pro Lys Gln Met Pro Leu Ser Gln Val
           675                 680                 685

## Description

### STATEMENT REGARDING FEDERALLY SPONSORED RESEARCH OR DEVELOPMENT

The present invention was generated at least in part with funds from National Institutes of Health NIH, NIDDK DK52596; the Veterans Administration (VA Merit Review Funding); and intramural National Cancer Institute funds. The United States Government may have certain rights in the invention.

### FIELD OF THE INVENTION

The present invention is directed to fields of medicine, biochemistry, cell biology, and chemistry. More specifically, the present invention addresses a novel compound and derivatives thereof having growth inhibitory activity. The present invention also relates to the treatment of any disease involving uncontrolled cell proliferation, such as cancer.

### BACKGROUND OF THE INVENTION

Approximately one million people in the United States suffer from the bladder disorder interstitial cystitis, which is a chronic painful urinary bladder condition characterized by thinning or ulceration of the bladder epithelial lining (Curhan *et al.*, 1999).

Cystoscopic abnormalities seen in the bladder of patients with this disorder include petechial hemorrhages called "glomerulations" and ulcers that extend into the lamina propria (Hunner's ulcers) (Johansson and Fall, 1990; Skoluda *et al.*, 1974). The most consistent histologic abnormalities include denudation or thinning of the bladder epithelium to 1-2 cell layers (Johansson and Fall, 1990; Skoluda *et al.*, 1974; Tomaszewski *et al.*, 2001). These findings suggest that interstitial cystitis may be caused by an inhibition of normal bladder epithelial cell proliferation, resulting in a loss of epithelial barrier integrity with subsequent exposure of sensory nerve cells in the bladder wall to urinary constituents. However, the pathogenesis of interstitial cystitis is heretofore unknown.

The isolation of an antiproliferative factor ("APF") peptide that is made uniquely by bladder epithelial cells from interstitial cystitis patients (Keay *et al.*, 2001; Keay *et al.*, 2000) and profoundly inhibits normal bladder epithelial cell growth (Keay *et al.*, 2003) was previously described. U.S. Patent No. 5,962,645 teaches a purified human antiproliferative factor (APF) isolated from the urine of patients with interstitial cystitis wherein the APF is characterized by a molecular weight of about 1.7 kDa determined by mass spectrometry on a sample in an aqueous acetonitrile solution and a pI range of about 1.38-3.5, and the APF is capable of inhibiting normal human bladder epithelial (HBE) and bladder carcinoma cell proliferation. Picomolar quantities of HPLC-purified APF were able to induce several changes in normal bladder epithelial cells *in vitro*, including significantly decreased rates of proliferation (Keay *et al.*, 2003) and decreased production of a growth factor required for log-phase growth of bladder epithelial cells (heparin-binding epidermal growth factor-like growth factor, or HB-EGF) (Keay *et al.*, 2000; Keay *et al.*, 2003).

HB-EGF has been previously described (for example, see U.S. Patent No. 5,811,393). U.S. Patent No. 6,156,522 describes a method for diagnosing interstitial cystitis in a subject suffering from bladder dysfunction, said method comprising the steps of (a) measuring the levels of HB-EGF-like growth factor in the urine sample of the subject; and (b) comparing said level with normal levels, wherein decreased levels of heparin-binding epidermal growth factor-like growth factor, as compared to levels of heparin-binding epidermal growth factor-like growth factor in a normal population, indicates the presence of interstitial cystitis. U.S. Patent 6,232,289 teaches a method for enhancing bladder epithelial cell proliferation in a subject in need thereof, said method comprising administering to the subject HB-EGF, in an amount effective to enhance bladder epithelial cell proliferation. U.S. Patent 6,376,197 teaches a method for diagnosing a condition such as interstitial cystitis associated with inhibited bladder epithelial cell proliferation comprising the steps of determining the level of epidermal growth factor in urine from the subject; and comparing said level with normal level, according to the following criterion: increased level of epidermal growth factor, as compared to level of epidermal growth factor in a normal population, indicates the presence of the condition.

Microarray analysis indicated that APF can also induce changes in the pattern of cellular gene expression toward a more differentiated phenotype (Keay *et al.*, 2003). Identification of this factor is therefore important for determining its potential role in the pathogenesis of interstitial cystitis and establishing its utility as a biomarker for this disease.

Preliminary characterization of APF indicated that it was a low molecular weight, relatively heat stable peptide (Keay *et al.*, 2000). APF is found in minute quantities in both patient urine specimens and explanted patient bladder cell supernatants, making conventional methods of structural analysis, such as NMR spectroscopy, unfeasible. As described herein, the complete characterization of this potent growth inhibitor provides a novel structure not previously disclosed. The complete characterization was made using a combination of techniques including mass spectrometry, lectin affinity chromatography, enzymatic analysis, and total synthesis.

Confirmation of the structure of APF was provided using microcapillary liquid chromatography of native and synthetic APF derivatives, as well as by demonstration of synthetic APF's ability to regulate growth factor production and bladder epithelial cell proliferation. Additional evidence of APF's identity was provided by identification of mRNA that bound to a probe for the frizzled 8-protein segment in cells from interstitial cystitis patients but not controls, as well as by binding of rabbit antibodies raised against synthetic APF to purified native APF from supernatants of bladder epithelial cells of interstitial cystitis patients.

Another factor considered to be a cell cycling inhibitory factor is described in U.S. Patent No. 5,916,871, wherein the factor comprises a sialylated glycopeptide preferably having a molecular weight much larger (e.g. 18 kD or 66 kD) than APF and that inhibits the G1 phase (but not G2) of the cell cycle. This previously described factor also had a carbohydrate component that accounted for less than 10% of its total mass, whereas APF in its native form has carbohydrate that accounts for 44% of its total mass.

### BRIEF SUMMARY OF THE INVENTION

The present invention concerns an antiproliferative factor (APF).

The APF molecule comprises a glycopeptide that inhibits in part or in full cell proliferation and/or slows cell proliferation, for example. The cell being proliferated may be an epithelial cell. APF may be considered a negative growth factor, negative growth regulator, or toxin. Although APF is a urinary bladder-specific APF or a urinary bladder epithelial cell-specific APF, it is also useful for other tissues, such as to inhibit the proliferation of non-bladder cells, including fibroblasts or prostate cells, for example. Because interstitial cystitis patients have the same alterations in the serum levels of HB-EGF and EGF in serum as seen in urine specimens (Keay *et al.*, 2000), APF may produced in tissues other the urinary bladder.

As delineated in Rashid *et al.* (2004), APF is a cell-cycle modulator. That is, explanted cells from normal bladder biopsy specimens were exposed to APF, which increased significantly the proportion of tetraploid and hypertetraploid cells compared to controls. Thus, exposure of a cell to APF provides a block in cell cycling in the G2 and/or M phase cell cycle block and/or the production of polyploidy. As such, APF affects cell cycle distribution, which in particular embodiments contributes at least in part to the pathogenesis of bladder disorders such as interstitial cystitis, for example through disruption of normal urothelial proliferation and repair processes. In further embodiments, exposure of one or more cells to APF results in inhibition of proliferation of the one or more cells, which may comprise a cell cycle block at any point in the cell cycle, although in particular embodiments the block is primarily in G2 or M phase. In particular embodiments, removal of APF permits the cell cycle to resume.

APF is a sensitive and specific biomarker for a bladder disorder such as interstitial cystitis (IC), and in a specific embodiment it plays a critical role in the pathogenesis of IC by profoundly inhibiting bladder cell proliferation, such as *via* regulation of gene expression (such as by increased E-cadherin production, for example) and alterations in the production of specific growth factors (such as HB-EGF and EGF, for example). More specifically, the inhibition of bladder cell proliferation by APF involves inhibition of HB-EGF production; stimulation of cellular EGF, E-cadherin, arylsulfatase A, phosphoribosylpyrophosphate synthetase-associated protein 39, or SWI/SNF complex 170 kDa subunit gene expression; or inhibition of cellular putative tRNA synthetase-like protein, vimentin, neutral amino acid transporter B, possible GTP-binding protein, alpha 1 catenin, alpha 2 integrin, cyclin D1 and JNK or ribosomal protein L27a gene expression.
The present invention provides a synthetic glycopeptide which consists of (a) one to six sugar moieties and (b) a peptide whose sequence consists of SEQ ID NO:1, 3, 4, 5 or 6.

In one specific aspect of the invention, APF is an acidic, heat stable sialoglycopeptide comprising 9 amino acid residues (such as, for example, TVPAAVVVA, SEQ ID NO:1; SVPAAVVVA, SEQ ID NO:3; TVPAAVVLA, SEQ ID NO:4; or SLPAAVVVA, SEQ ID NO:5) covalently linked through the N-terminal threonine, serine, or cysteine, for example, to an N-acetylgalactosamine or N-acetylglucosamine residue that is linked *via* an α or β configuration to galactose, and sialylated on the galactose moiety *via* 2,3 linkage. The anomeric configuration of the glycosyl bond is alpha in particular embodiments, although it may be beta in alternative embodiments.

The present invention encompasses synthetic APF glycopeptides. Like native APF, synthetic APF profoundly inhibited bladder epithelial cell proliferation (IC₅₀=0.4 nM) and HB-EGF production, while stimulating EGF production. Synthetic APF glycopeptides also inhibited proliferation of bladder carcinoma cells that are sensitive to native APF. In specific embodiments of the invention, nonsialylated synthetic APF glycopeptides comprise functional activity as their sialylated counterparts, although nonglycosylated synthetic peptide and the beta anomer of glycosylated synthetic APF comprised less desirable activity.

In one embodiment, the APF compound comprises a sugar moiety having one or more sugars, wherein the sugars are referred to herein as a first sugar, a second sugar, a third sugar, and so forth. Although any of the sugars may be covalently linked to a peptide, for example, in specific embodiments the third sugar is covalently linked to a peptide, such as one having a sequence as set forth in SEQ ID NOS:1, 3, 4, or 5. The sugar moiety may include naturally-occurring sugars, synthetic sugars, derivatives thereof including sugar mimetic components, and/or any combination thereof.

The compounds of the present invention comprise a peptide moiety, which may be characterized by having a terminal subunit having a polar chemical characteristic and/or a heteroatom therein. The subunits of the peptide may include naturally-occurring amino acid residues, unnatural amino acids, derivatives of amino acids, such as methylated amino acids, peptidomimetic components and/or any combination thereof.

In preferred embodiments, the sugar molecule includes one or more of a sialic acid, galactose, glucose, N-acetylglucosamine, and/or N-acetylgalactosamine, for example. In certain embodiment, the sialic acid molecule is covalently linked to the galactose or glucose through a (2, 3), a (2, 6), a (2, 8), and/or a (2,9) linkage. A skilled artisan is aware of the nomenclature used in sugar/carbohydrate chemistry to identify the atom at the locations specified. Alternatively, the galactose or glucose is covalently linked to the N-acetylgalactosamine or N-acetylglucosamine molecule through a 1->3, a 1->6 or a 1->4 linkage. In a further preferred embodiment, the N-acetylgalactosamine or N-acetylglucosamine sugar molecule is linked to the hydrophobic moiety in the alpha configuration.

Oligonucleotides that encode the nonapeptide of SEQ ID NO:1 or biologically functional derivatives thereof, such as the peptides of SEQ ID NO:3, SEQ ID NO:4, or SEQ ID NO:5, for example, and/or precursors of APF are also disclosed herein. An exemplary oligonucleotide that encodes SEQ ID NO:1 is SEQ ID NO:2. However, given the limited choices of triplet nucleotides per given codon for a particular amino acid, a skilled artisan recognizes that a polynucleotide encoding an APF peptide, exemplary embodiments of which include SEQ ID NO:1, SEQ ID NO:3, SEQ ID NO:4, SEQ ID NO:5, or SEQ ID NO:6 are limited in number. This is particularly true given the further subset of codons available for encoding hydrophobic amino acids, which are preferably comprised in at least part of the peptide moiety of the APF molecule.

Compositions comprising the APF compounds of the present invention are contemplated. The compositions may further comprise a delivery agent, such as a liposome; encapsulated cell; conjugated molecules, such as antibodies (Safavy *et al.*, 2003), other peptides, and a variety of non-peptide conjugates (including folate and polyethylene glycol; Aronov *et al.*, 2003); drugs, such as geldanamycin (Mandler *et al.*, 2004) or insulin (Ou *et al.*, 2003); liposomes (Heath and Martin, 1986); lactosaminated human albumin (Di Stefano *et al.*, 2003); polyethylene glycol (PEG) (Aronov *et al.*, 2003); nanoparticles, such as colloidal gold; or other molecules that bind to cell surface receptors to facilitate cellular interaction with or uptake of APF.

Antibodies to synthetic APF glycopeptides of the invention may be used in a method that detects a bladder disorder in an individual by assaying for an APF molecule. The bladder disorder is interstitial cystitis. This provides the advantage of being able to assay for an APF molecule by non-invasive means, although in alternative embodiments invasive means may be used. A sample may be obtained from the individual and assayed directly or indirectly for the APF molecule. The sample may comprise urine, plasma, or other bodily fluid specimen, such as whole blood, feces, saliva, nipple aspirate, mucus, or sweat. A urine sample may be obtained following voiding from the individual, and it may be obtained *via* a catheter.

Diagnostic methods comprise detection of the peptide or a combination of peptide and sugar moieties of the APF molecule. Assaying steps may comprise introduction to a sample an antibody directed to an epitope of the peptide moiety of an APF molecule.

The APF compounds of the invention may be used in to methods of treating epithelial hyperplasia or malignancies of epithelial origin comprising administering an effective amount of APF or derivatives thereof to a patient in need of such treatment.

The APF compounds of the present invention may be used in methods of treating fibroblast hyperplasia or malignancy comprising administering an effective amount of APF or derivatives thereof to a patient in need of such treatment.

The APF compounds of the present invention may be used in methods of treating lymphoreticular malignancies or solid tumors comprising administering an effective amount of APF or derivatives thereof to a patient in need of such treatment.

The present invention provides a glycopeptide or composition of the invention for use in a method of treating cancer or a hyperplasia. Such a method may comprise administering an effective amount of APF or derivatives thereof to a patient in need of such treatment. Any kind of cancer may be treated, such as bladder, lung, breast, prostate, brain, stomach, colon, spleen, liver, pancreatic, melanoma, head and neck, thyroid, and so forth. In specific embodiments, though, the invention is useful for treating bladder or prostate cancer, comprising co-administering an effective amount of APF to a patient in need of such treatment. The APF may improve, facilitate, or assists in overcoming resistance or improving sensitivity to a cancer therapy selected from the group of chemotherapy, radiotherapy, surgery gene therapy, and/or immunotherapy.

The APF compounds may be used to generate antibodies thereto, in which the antibodies may be used for treating interstitial cystitis. Antibodies may be generated against a peptide moiety of an APF composition. For example, antibodies may be generated against a peptide moiety of APF, such as one comprising SEQ ID NO:1, SEQ ID NO:3, SEQ ID NO:4, SEQ ID NO:5, or SEQ ID NO:6, for example. The antibodies that are generated may be polyclonal or monoclonal. As used herein, the term "antibody" is intended to refer broadly to any immunologic binding agent such as IgG, IgM, IgA, IgD and IgE. Generally, IgG and/or IgM are preferred because they are the most common antibodies in the physiological situation and because they are most easily made in a laboratory setting.

The term "antibody" may also be used to refer to any antibody-like molecule that has an antigen binding region, and includes antibody fragments such as Fab', Fab, F(ab')2, single domain antibodies (DABs), Fv, scFv (single chain Fv), and the like. The techniques for preparing and using various antibody-based constructs and fragments are well known in the art. Means for preparing and characterizing antibodies are also well known in the art (See, e.g., Antibodies: A Laboratory Manual, Cold Spring Harbor Laboratory, 1988). Antibodies are prepared by immunizing an animal with a composition in accordance with the present invention and collecting antisera from that immunized animal.

As is also well known in the art, the immunogenicity of a particular immunogen composition can be enhanced by the use of non-specific stimulators of the immune response, known as adjuvants. Suitable adjuvants include all acceptable immunostimulatory compounds, such as cytokines, chemokines, cofactors, or toxins, for example. Adjuvants that may be used include IL-1, IL-2, IL-4, IL-7, IL-12, γ-interferon, GMCSP, BCG, aluminum hydroxide, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion is also contemplated. MHC antigens may even be used. Exemplary, often preferred adjuvants include complete Freund's adjuvant (a non-specific stimulator of the immune response containing killed Mycobacterium tuberculosis), incomplete Freund's adjuvants and aluminum hydroxide adjuvant.

The present invention provides a synthesized composition comprising a urinary bladder antiproliferative factor having one to six sugar moieties, wherein at least one sugar moiety is linked to a hydrophobic moiety. The composition may be further defined as a urinary bladder epithelial cell antiproliferative factor. The molecular weight of the factor may be less than about 3000 Daltons. The hydrophobic moiety comprises a peptide moiety, which may be linear, cyclical, or branched. The APF composition comprises a sialoglycopeptide, in specific embodiments, although in alternative embodiments it comprises a glycopeptide. The peptide has homology to at least part of a frizzled polypeptide, such as having homology to at least part of a transmembrane domain of frizzled 8.

The composition is further defined as consisting of one to six sugar moieties; and a peptide moiety, wherein one of the amino acid residues is a linking amino acid, and wherein the peptide is linked to at least one of the sugar moieties at a heteroatom of the linking amino acid, which may be polar, such as a serine, a threonine, a cysteine, a lysine, an arginine, or a tyrosine. Thus, it is contemplated that the heteroatom linked to the sugar moiety is an oxygen, nitrogen and/or a sulfur atom. In further specific embodiments, the composition comprises three sugar residues and nine amino acids, wherein the linking amino acid is a serine or a threonine. In yet other specific embodiments, the composition comprises two sugar residues and nine amino acids

The sugar moiety comprises a naturally occurring sugar, a synthetic sugar, a derivative of a naturally occurring sugar, or a derivative of a synthetic sugar. More specifically, at least one sugar moiety is an amino sugar such as a sialic acid (N-acetylneuraminic acid) molecule, and in some embodiments, and in some embodiments, the amino sugar is linked to at least another (second) sugar *via* a (2,3) linkage, a (2,6) linkage, a (2,8) linkage, or a (2,9) linkage. The linkage between at least one sugar moiety and a peptide moiety is a covalent linkage; the linkage between a sugar moiety and a lipid moiety is a covalent linkage; and other linkages described herein may be covalent.

In specific embodiments involving more than one sugar moiety, the linkage between one sugar moiety and another sugar moiety is a 1→3 linkage, a 1→4 linkage, or a 1→6 linkage. In other embodiments, the linkage between at least one sugar moiety and a hydrophobic moiety, such as a peptide or a lipid, is in the alpha or beta configuration.

The peptide moiety of the composition is further defined as comprising a naturally occurring amino acid, an unnatural amino acid, a derivative of a naturally occurring amino acid, a derivative of an unnatural amino acid, a modified amino acid, a backbone-modifying amino acid, or a mixture thereof The peptide moiety is further defined as comprising one or more backbone-modifying amino acids that comprise reduced peptide bonds. Modified amino acids may be further defined as a methylated amino acid, an acetylated amino acid, a beta amino acid, or an amino acid mimetic.

The peptide moiety is nine amino acids in length, and consists of SEQ ID NO:1, SEQ ID NO:3, SEQ ID NO:4, SEQ ID NO:5 or SEQ ID NO:6.

An APF composition of the present invention may be further defined as comprising a label, such as a fluorescent moiety, a colorimetric moiety, or a radioactive moiety. In certain embodiments, the label is attached to at least one of the one or more sugar moieties, such as sialic acid, glucose, galactose, N-acetylgalactosamine or N-acetylglucosamine. Alternatively, the label is attached any suitable atom in a peptide, such as within at least one of the amino acids making up the peptide moiety, such as at a heteroatom in a serine, threonine, or cysteine amino acid subunit, or attached to the carboxyl group at the carboxyl end of the peptide.

In specific embodiments of the present invention, the APF composition is further defined as:

(a) Sialic acid-galactose-Nacetylgalactosamine-threonine-valine-proline-alanine-alanine-valine-valine-valine-alanine;

(b) Sialic acid-galactose-Nacetylglucosamine-threonine-valine-proline-alanine-alanine-valine-valine-valine-alanine; or

(c) Sialic acid-galactose-Nacetylglucosamine-serine-leucine-proline-alanine-alanine-valine-valine-valine-alanine.

As described further in the Examples herein, the APF compositions of the present invention are alternatively defined as any one of (a), (b) or (c) without a sialic acid molecule.

The composition of (a) may be further defined as having one (or one or more) of the following: the sialic acid is linked to galactose *via* a 2,3 linkage; the galactose is linked to the N-acetylgalactosamine *via* a 1,3 linkage; and/or the N-acetylgalactosamine is linked to threonine *via* an O linkage in an alpha configuration.

The composition of (b) may be further defined as having one (or one or more) of the following: the sialic acid is linked to galactose *via* a 2,3 linkage; the galactose is linked to the N-acetylglucosamine *via* a 1,4 linkage; and/or the N-acetylglucosamine is linked to threonine *via* an O linkage in an alpha configuration.

The composition of (c) may be further defined as having one (or one or more) of the following: the sialic acid is linked to galactose *via* a 2,3 linkage; the galactose is linked to the N-acetylglucosamine *via* a 1,4 linkage; and/or the N-acetylglucosamine is linked to serine *via* an O linkage in an alpha configuration.

In some embodiments of the present invention, the composition further comprises a delivery vehicle, such as a liposome, a cell, a conjugated molecule, a nanoparticle, or a mixture thereof. The conjugated molecule may comprise an antibody, a peptide, folate, polyethylene glycol, a drug, a liposome, a microsphere, or lactosaminated human albumin. The nanoparticle may comprise colloidal gold.

The composition may be comprised in a pharmaceutically acceptable excipient. The composition may also reversibly arrest cell proliferation. In specific embodiments, the composition is further defined as comprising activity for arresting cell cycling primarily in G2 or M phase or both.

Also described is an isolated peptide selected from the group consisting of SEQ ID NO:1; SEQ ID NO:3; SEQ ID NO:4; SEQ ID NO:5, or a functional derivative thereof. The functional derivative thereof may be further defined as comprising a conservative substitution at one or more amino acids of the peptide. The conservative substitution may be further defined as a substitution at serine, threonine, proline, or a combination thereof. The conservative substitution may comprise a hydrophobic conservative substitution, such as a substitution at one or more alanines, one or more valines, one or more prolines; or a combination thereof. A function of such peptides may be for use as a standard in a kit (such as to quantify APF in samples; as an antiproliferative factor; and/or as a means for inducing antibody production.

Also described is an isolated polynucleotide encoding SEQ ID NO:1, which may be further defined as SEQ ID NO:2.

The polynucleotides may be further defined as having one or more of the following: a codon for threonine selected from the group consisting of ACA, ACC, ACG, and ACU; a codon for one or more valines selected from the group consisting of GUA, GUC, GUG, and GUU; a codon for proline selected from the group consisting of CCA, CCC, CCG, and CCU; and a codon for one or more alanines selected from the group consisting of GCA, GCC, GCG, and GCU.

Also described is an isolated polynucleotide encoding SEQ ID NO:3, such as one further defined as having one or more of the following: a codon for serine selected from the group consisting of AGC, AGU, UCA, UCC, UCG, and UCU; a codon for one or more valines selected from the group consisting of GUA, GUC, GUG, or GUU; a codon for proline selected from the group consisting of CCA, CCC, CCG, or CCU; and a codon for one or more alanines selected from the group consisting of GCA, GCC, GCG, or GCU.

Also described is an isolated polynucleotide encoding SEQ ID NO:4, such as one further defined as having one or more of the following: a codon for threonine selected from the group consisting of ACA, ACC, ACG, and ACU; a codon for one or more valines selected from the group consisting of GUA, GUC, GUG, and GUU; a codon for proline selected from the group consisting of CCA, CCC, CCG, and CCU; a codon for one or more alanines selected from the group consisting of GCA, GCC, GCG, and GCU; and a codon for leucine selected from the group consisting of UUA, UUG, CUA, CUC, CUG, and CUU.

Also described is an isolated polynucleotide encoding SEQ ID NO:5, such as one further defined as having one or more of the following: a codon for serine selected from the group consisting of AGC, AGU, UCA, UCC, UCG, and UCU; a codon for leucine selected from the group consisting of UUA, UUG, CUA, CUC, CUG, and CUU; a codon for proline selected from the group consisting of CCA, CCC, CCG, and CCU; a codon for one or more alanines selected from the group consisting of GCA, GCC, GCG, and GCU; and a codon for one or more valines selected from the group consisting of GUA, GUC, GUG, and GUU.

In additional embodiments of the present invention, there is a kit, comprising the APF composition housed in a suitable container. In specific embodiments, the antiproliferative factor is a urinary bladder antiproliferative factor. The peptide may comprise one or more hydrophobic amino acids.

Antibodies may bind immunologically to an epitope of SEQ ID NO:1 or a glycopeptide derivative thereof and/or the peptide comprises an epitope of SEQ ID NO: and is about 2 to 15 amino acids in length; the antibody binds immunologically to an epitope of SEQ ID NO:3 or a glycopeptide derivative thereof and/or the peptide comprises an epitope of SEQ ID NO:3 and is about 2 to 15 amino acids in length; the antibody binds immunologically to an epitope of SEQ ID NO:4 or a glycopeptide derivative thereof and/or the peptide comprises an epitope of SEQ ID NO:4 and is about 2 to 15 amino acids in length; the antibody binds immunologically to an epitope of SEQ ID NO:5 or a glycopeptide derivative thereof and/or the peptide comprises an epitope of SEQ ID NO:5 and is about 2 to 15 amino acids in length; and/or the antibody binds immunologically to an epitope of SEQ ID NO:6 or a glycopeptide derivative thereof and/or the peptide comprises an epitope of SEQ ID NO:6 and is about 2 to 15 amino acids in length.

Antibodies include monoclonal antibodies; polyclonal antibodies, or both. The antibody may be labeled.

A kit may comprise a detection means for detecting APF compositions in a sample and includes antibodies, markers such as fluorescent reporter molecules, and/or capture molecules that target a sugar or peptide moiety of the composition, in additional embodiments.

Antibodies to the synthetic glycopeptides of the present invention, may be used in diagnosing a bladder disorder in subject. Said diagnosis may comprises the step of assaying for an APF composition. In specific embodiments, the bladder disorder is interstitial cystitis. The assaying step is non-invasive or invasive to the individual. The method may be further defined as obtaining a sample from the individual; and assaying said sample for the presence of the APF composition. The sample may comprise urine, plasma, serum, tissue, or a mixture thereof from the individual. The assaying step may comprise detection of one or more sugar moieties of an APF composition. The assaying step may comprise detection of the hydrophobic moiety of the APF composition. The hydrophobic moiety of the composition may comprise a peptide moiety and the assaying step is further defined as comprising introduction to the sample of an antibody directed to an epitope of a peptide, sugar, or glycopeptide moiety of an APF composition. The assaying method may comprise ELISA Western blot, immunoblot, radioimmunoassay, immunohistochemistry, or other antibody-based detection methods, including slot blots, dot blots and so forth. The assaying method may comprise *in situ* hybridization or other means for detecting messenger mRNA for APF, for example. The assaying method may comprise mass spectrometry, nuclear magnetic resonance, or other non-immunological methods for detecting peptide species.

The invention provides a glycopeptide of the invention for use in a method of treating a cancer or a hyperplasia. The method of treating cancer may comprise the step of administering a therapeutically effective amount of an APF composition. In specific embodiments, the cancer comprises an epithelial cancer, such as bladder cancer or prostate cancer. In an additional specific embodiment, the method further comprises an additional cancer therapy, such as surgery, chemotherapy, radiation, gene therapy, immunotherapy, or a combination thereof.

The method of treating a hyperplasia may comprise the step of administering a therapeutically effective amount of an APF composition. In a specific embodiment, the method further comprises an additional therapy for the hyperplasia, such as an epithelial hyperplasia or a fibroblast hyperplasia.

### BRIEF DESCRIPTION OF THE DRAWINGS

For a more complete understanding of the present invention, reference is now made to the following descriptions taken in conjunction with the accompanying drawings.:

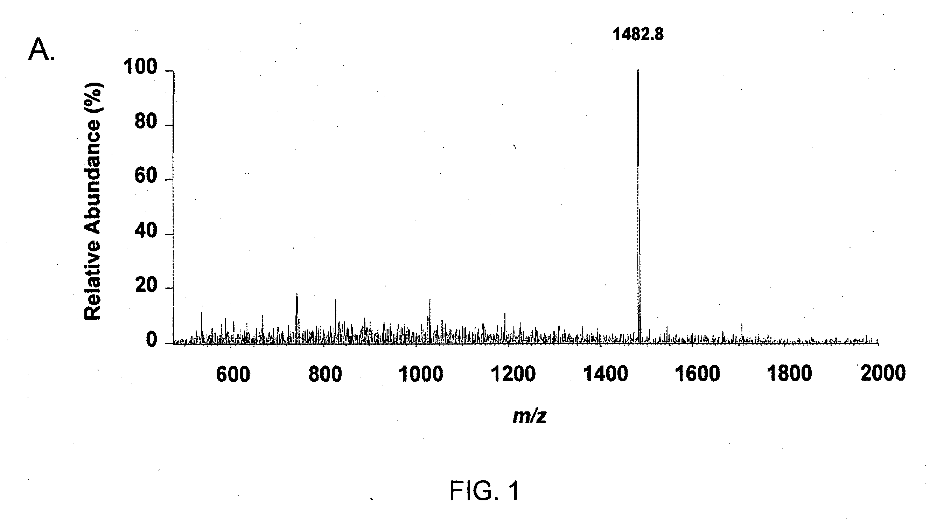

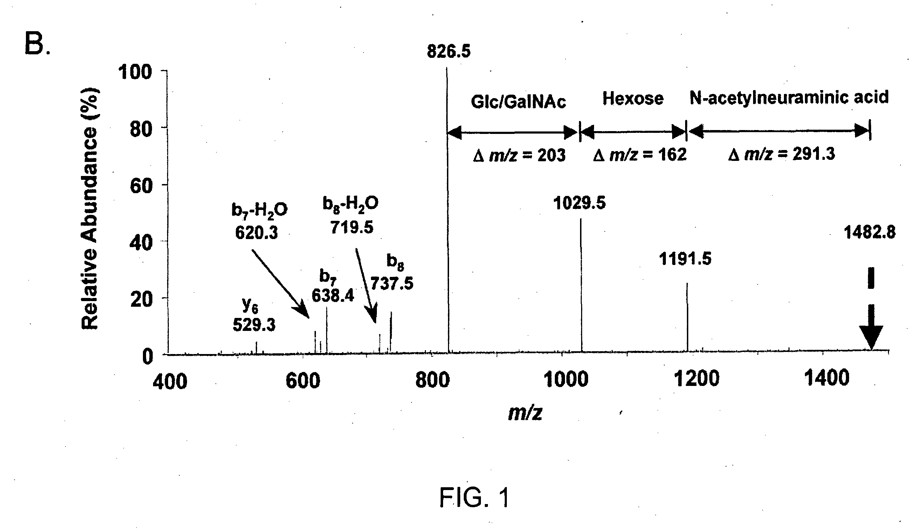

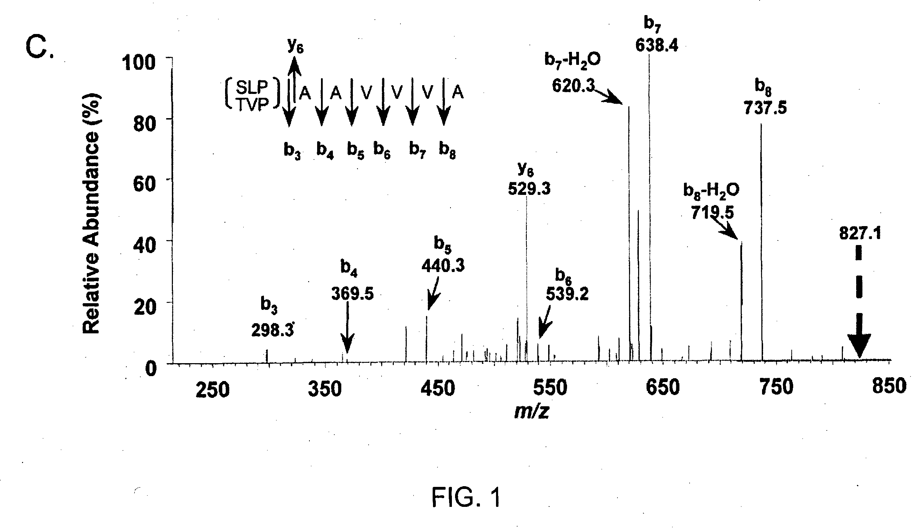

FIGS. 1A-1C show ion trap mass spectrometric analysis of APF. In FIG. 1A, there is molecular mass analysis of the active peak from microcapillary fractionation of HPLC-purified APF. FIGS. 1B and 1C provide analysis following successive fragmentation of predominant species by collision-induced dissociation.

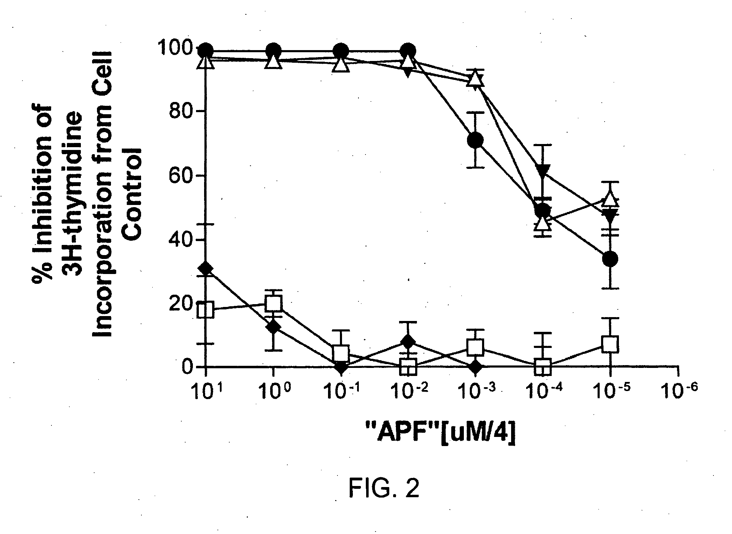

FIG. 2 demonstrates antiproliferative activity of APF peptide and glycosylated derivatives. Inhibition of primary normal bladder epithelial cell ³H-thymidine incorporation by synthetic APF and its derivatives. Equimolar quantities of the peptide backbone alone (- 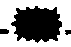 -), N-acetyllactosamine-α-O-Thr derivative (closed -▼-), sialylated N-acetyllactosamine-α-O-Thr derivative (-Δ-), N-acetyllactosamine-β-O-Thr derivative (closed -◆-), and Galβ1-3GalNAc-α-O-Thr derivative (closed -●-), were applied to normal bladder epithelial cells, and ³H-thymidine incorporation was determined and compared to incorporation in cells grown in medium containing buffer alone. All specimens were assayed in triplicate in 2-3 repeated experiments; data are expressed as the mean inhibition of incorporation, and vertical lines indicate standard error of the mean.

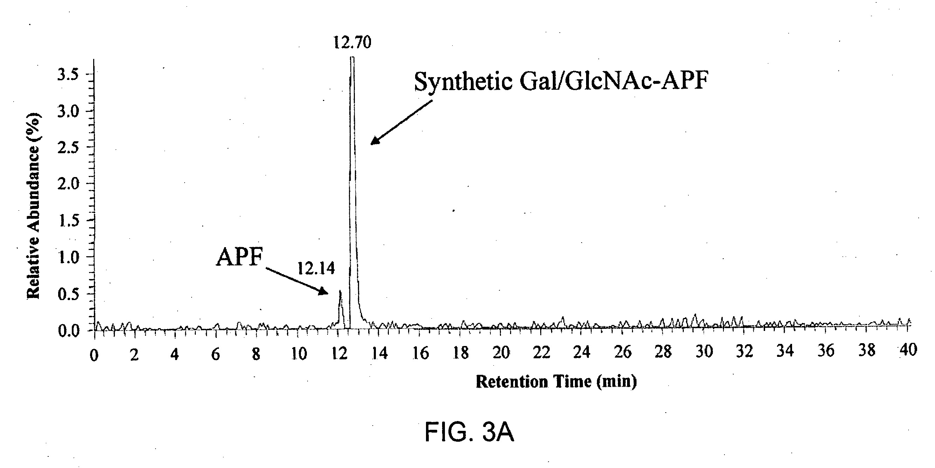

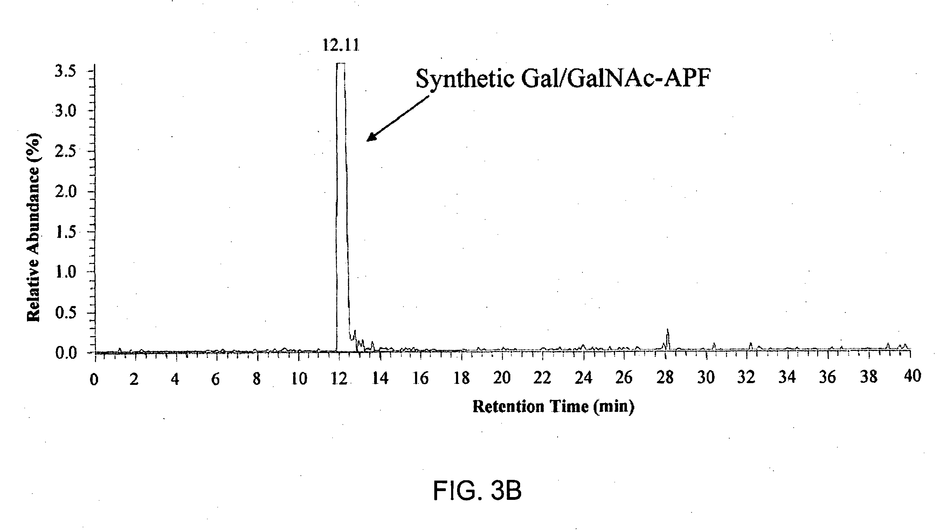

FIGS. 3A and 3B provide microcapillary reversed-phase liquid chromatography of native APF, and synthetic GlcNAc (also referred to as Galβ1-4GlcNAcα-O-TVPAAVVVA) and GalNAc (also referred to as Galβ1-3GalNAcα-O-TVPAAVVVA, in some embodiments) derivatives. Neuraminidase-treated APF was injected along with either synthetic GalGlcNAc-containing APF or synthetic GalGalNAc-containing APF and their relative mobilities on C18 determined.

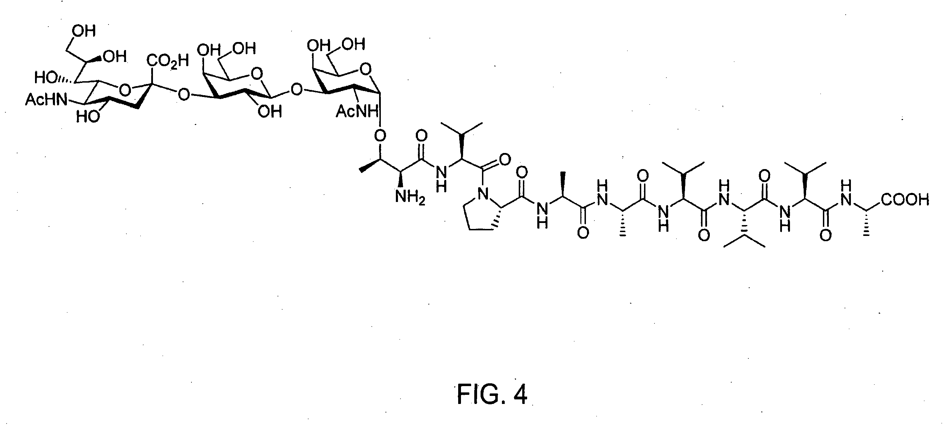

FIG. 4 illustrates one embodiment of the structure of APF, Galβ1-3GalNAcα-O-TVPAAVVVA.

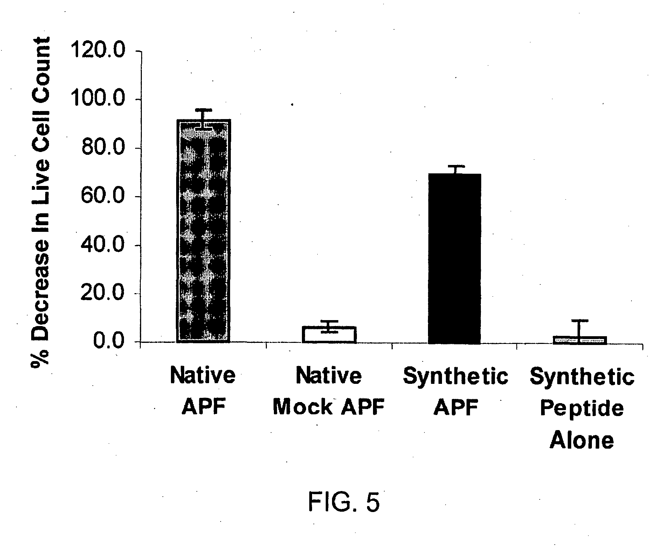

FIG. 5 shows inhibition of T24 cell proliferation by native and synthetic APF. Native APF (estimated concentration 3-5 µg/ml based on absorbance at 260 nm), an equivalent volume of Mock APF, synthetic GalGalNAc APF (3.0 µg/ml of glycopeptide), or an equimolar amount of peptide backbone alone were compared for their ability to inhibit cell proliferation by determining live cell count after 48 hours using trypan blue exclusion. All specimens were assayed in triplicate; data are expressed as the mean decrease in cell count, and vertical lines indicate standard error of the mean.

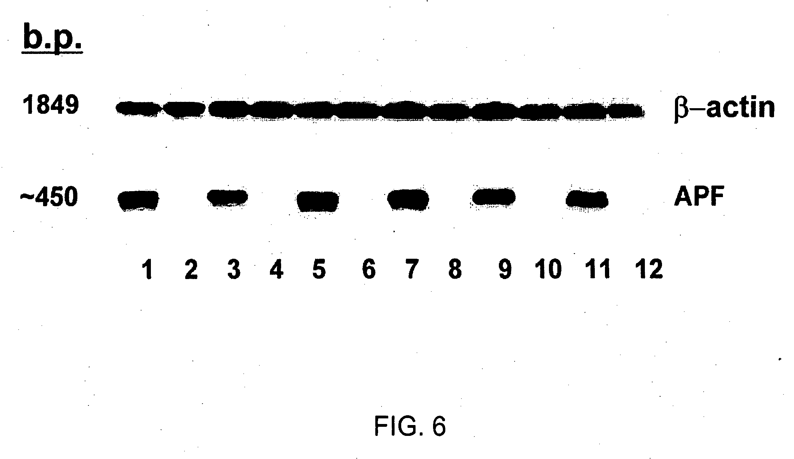

FIG. 6 provides Northern blot analysis of mRNA encoding the APF nonapeptide. Total RNA was extracted from explanted bladder epithelial cells from 6 patients with interstitial cystitis (lanes 1, 3, 5, 7, 9, and 11) and their age-, race-, and gender matched asymptomatic controls (lanes 2, 4, 6, 8, 10, and 12). The membrane was then incubated with DIG-labeled probes for APF mRNA and beta actin mRNA and developed using a chemiluminescent substrate.

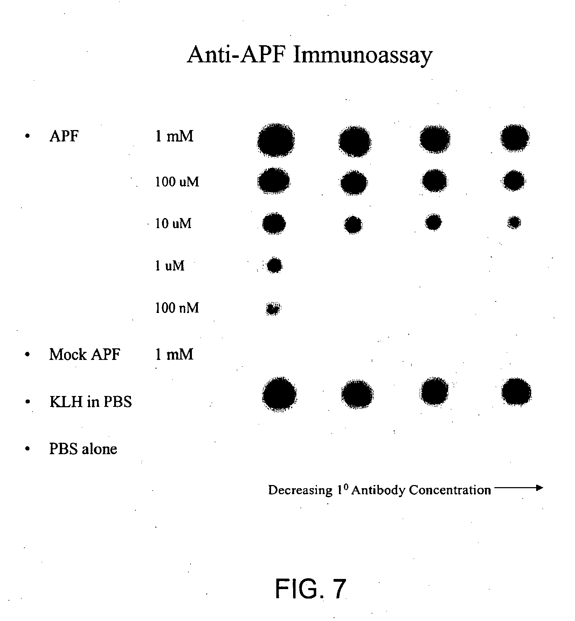

FIG.7 demonstrates dose-dependent binding to HPLC-purified native APF of purified rabbit antibodies raised against synthetic APF.

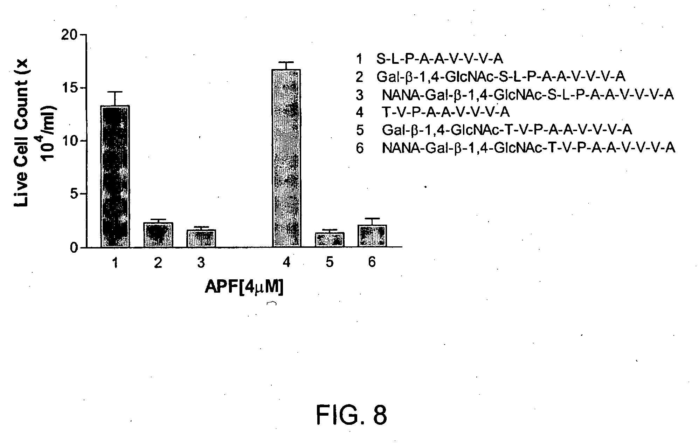

FIG. 8 demonstrates antiproliferative activity against normal human bladder epithelial cells of 2 different peptides (#1, corresponds to SEQ ID NO:1, and #4, corresponds to SEQ ID NO:5) and their sugar derivatives, having two (#2 and #5, each unsialyated) and three (#3 and #6) sugar moieties.

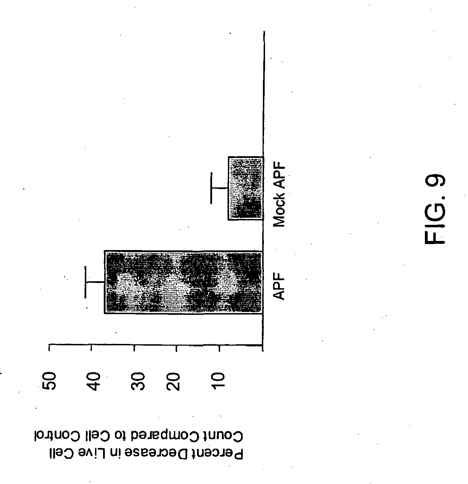

FIG. 9 shows APF inhibitory activity against LNCaP prostate cancer cells *in vitro.*

### DETAILED DESCRIPTION OF THE INVENTION

### I. Definitions

As used herein the specification, "a" or "an" may mean one or more. As used herein in the claim(s), when used in conjunction with the word "comprising", the words "a" or "an" may mean one or more than one. As used herein "another" may mean at least a second or more.

The term "alpha configuration" α as used herein refers to structural relationships in carbohydrate chemistry, wherein the anomeric group is in the axial configuration when the conformational formulation of the pyranose ring is used. Conversely, the term "beta configuration" β refers to that arrangement in which the anomeric group is equatorial.

The term "antiproliferative factor" as used herein refers to a molecule comprised of one or more sugar moieties and a hydrophobic moiety, wherein the molecule is characterized by the ability to inhibit cell proliferation. In specific embodiments, the inhibiting activity comprises inhibiting epithelial cell proliferation, such as bladder epithelial cell proliferation. The hydrophobic moiety is a peptide. In specific embodiments, the hydrophobic nature facilitates nonspecific association with a membrane, or specific or nonspecific interaction with a hydrophobic pocket of a membrane or cytoplasmic receptor, for example. In specific embodiments, the association with a membrane comprises insertion into a membrane. The membrane may be any kind of membrane, although in particular aspects of the invention it is a plasma membrane. In further specific embodiments, the peptide is hydrophobic in part and comprises enough hydrophobicity to facilitate association of APF with a membrane.

The term "backbone-modifying amino acid" is known in the art and is discussed as follows. Normal peptide or protein backbone is formed from polymerization of alpha amino acids, which have the amino group on the carbon adjacent to the carboxyl group. This produces a polymer in which the repeating unit is -[NHCH(R)C(O)]-, wherein R is the sidechain that makes each amino acid different, but the repeating unit that forms the back bone is as shown. If the amino group is moved to a different carbon, for example the beta-carbon of alanine, the backbone is no longer natural but still has many of the properties of peptides. Proteolytic enzymes do not recognize altered backbones. Beta-alanine or gamma-butyric acid are common backbone altering amino acids. They are not naturally found in peptides or proteins.

The term "bladder disorder" as used herein refers to an abnormal condition of the urinary bladder.

The term "conservative substitution" as used herein refers to replacing an amino acid in a peptide or polypeptide with a different amino acid of a similar chemical nature. For example, a nonpolar amino acid may be conservatively substituted with another nonpolar amino acid. In specific embodiments, a hydrophobic amino acid may be substituted with another hydrophobic amino acid.

The term "epithelial cancer" as used herein refers to a cancer in a tissue originating from epithelial cells of the tissue. For example, epithelial cancer may comprise urinary bladder; ureter; lung; heart; gastrointestinal tract (including the stomach, small intestine, large intestine, rectum, liver, pancreas and gall bladder); spleen; male reproductive tract, including the seminal vesicles, prostate, bulbourethral gland, vas deferens, epididymis, testes, and penis; female reproductive tract, including the ovaries, Fallopian tubes, uterus, cervix, and vagina; kidneys; adrenal glands; thymus; thyroid; skin; bone (including synovium); ocular tissues (including cornea, retina, and lens); cochlea; breast tissue; lymph nodes; oral mucosa (including gingival), salivary gland, parotid gland; and nasopharygeal mucosa (including sinus mucosa), for example.

The term "heteroatom" as used herein refers to an atom in an organic molecule that is other than carbon or hydrogen.

The term "hydrophobic" as used herein refers to lacking affinity for water.

The term "hydrophobic amino acid" as used herein refers to amino acids that are unable to form hydrogen bonds with water because they have no, or very small, electrical charges in their structure. In aqueous solution, hydrophobic amino acids disrupt the hydrogen bonding structure that is formed among water molecules, given that they are unable to contribute to it. Hydrophobic amino acids vary in size, and the majority of hydrophobic amino acids have a side chain that is purely hydrocarbon. Other things being equal, a larger hydrophobic side chain will be more strongly hydrophobic than a smaller one. Specific examples of hydrophobic amino acids include those that comprise aliphatic hydrocarbon side chains, such as alanine, valine, leucine, or isoleucine; aromatic side chains, such as phenylalanine or tryptophan; sulfur-comprising side chains, such as methionine; and/or imino acids, such as proline, for example. In particular embodiments, hydrophobic amino acids are considered to be alanine, valine, leucine, and isoleucine.

The term "hyperplasia" as used herein refers to the abnormal proliferation of normal cells in normal arrangement in a tissue.

The term "inhibitor of an antiproliferative factor" as used herein refers to an inhibitor of any APF as encompassed by the invention. The inhibitor may degrade APF, block its effect on cell proliferation, that it is a growth factor, or a combination thereof, for example. The inhibitor may comprise an antibody, small interfering RNA, oligonucleotide, small molecule, and so forth, for example.

The term "peptide" as used herein refers to a compound made up of a single chain of D- or L-amino acids or a mixture of D- and L-amino acids joined by peptide bonds. Generally, the peptides used herein contain at least two amino acid residues and are less than about 50 amino acids in length. D-amino acids are represented herein by a lower-case one-letter amino acid symbol (e.g., r for D-arginine), whereas L-amino acids are represented by an upper case one-letter amino acid symbol (e.g., R for L-arginine). The peptides may be cyclical, linear, branched, or a combination thereof.

"Polypeptide" as used herein refers to a polymer of at least two amino acid residues and which contains one or more peptide bonds. "Polypeptide" encompasses peptides and proteins, regardless of whether the polypeptide has a well-defined conformation.

The term "protein" as used herein refers to a compound that is composed of amino acids linked by peptide bonds, but in contrast to peptides, has a well-defined conformation, such as a well-defined three-dimensional conformation. Proteins, as opposed to peptides, generally consist of chains of 50 or more amino acids.

The term "purified" as used herein, is intended to refer to a glycoprotein composition, wherein the glycoprotein is purified to any degree relative to its naturally-obtainable state, i.e., in this case, relative to its purity within a eukaryotic cell or within a fluid such as cell medium or supernatant, or a biological fluid such as urine, serum, or plasma.

A "subunit," as used herein, is a monomeric unit that is joined to form a larger polymeric compound. The set of amino acids are an example of subunits. Each amino acid shares a common backbone (--C--C--N--), and the different amino acids differ in their sidechains. The backbone is repeated in a polypeptide. A subunit represents the shortest repeating pattern of elements in a polymer backbone. For example, two amino acids of a peptide are not considered one subunit because two amino acids would not have the shortest repeating pattern of elements in the polymer backbone.

The term "terminal amino acid" as used herein refers to the amino acid on the end of a linear peptide of an APF molecule, and may refer to a N-terminal amino acid or a C-terminal amino acid.

The terms "therapeutic agent", "therapeutic composition", and "therapeutic substance" refer, without limitation, to any composition that can be used to the benefit of an organism including but not limited to a mammalian organism. Such agents may take the form of ions, small organic molecules, peptides, proteins or polypeptides, glycopeptides (and other modified peptides), oligonucleotides, and oligosaccharides, for example.

The term "therapeutically effective amount" as used herein refers to the amount of a composition utilized alone or in combination with another compound for a therapeutic purpose that results in ameliorating at least one symptom or objective finding (sign) of the medical condition being treated. A skilled artisan recognizes that the invention is useful for providing less than a complete cure, so long as one or more symptoms or signs are alleviated. For example, in treating a bladder condition, a therapeutically effective amount would include the amount that facilitates any or all of the following: decrease (or by inhibition, an increase) in cell proliferation; reduction in pain, urgency, or frequency of urination; reduction in the amount, degree and/or intensity of thinning and/or ulceration of the bladder epithelial lining; and so forth.

The term "urinary bladder" as used herein refers to a distensible membranous sac that serves for the temporary retention of the urine of an individual. Normally it resides in the pelvis in front of the rectum, and it receives the urine from the two ureters, discharging it at intervals into the urethra through an orifice closed by a sphincter. The organ is lined with transitional hypoblastic epithelium.

The term "urinary bladder antiproliferative factor" as used herein refers to an antiproliferative factor as described herein that is associated primarily with the urinary bladder. It may be associated with a cell of the bladder, such as with an epithelial cell, and this then may be referred to as a "urinary bladder epithelial cell antiproliferative factor". The factor may be identified within one or more bladder epithelial cells or it may be identified following secretion from one or more cells, or both. In addition, or alternative to, the factor may be suspended in urine within a bladder or in urine excreted therefrom, or both. Such an association of the factor with the urinary bladder may permit the detection of the APF as diagnostic for a bladder condition, such as interstitial cystitis, for example. Although in some embodiments APF is located in the urinary bladder, in alternative embodiments the APF molecule is also associated with serum, plasma, or other tissue.

### II. The Present Invention

Negative growth factors are thought to be important for normal contact inhibition of eukaryotic cells, and a decrease in their activity has been associated with malignant cell growth. However, abnormally increased production of growth inhibitors has been implicated in only a few human disease states, including bone marrow failure (aplastic anemia and myelodysplastic syndrome), nontoxic goiter, chronic venous ulcers, ischemic manifestations associated with systemic sclerosis and delayed healing of gastric ulcers. In specific embodiments of the present invention, additional diseases are also caused by inappropriate inhibition of cell proliferation required for maintaining normal tissue integrity, which in solid tissues may be manifest as thinning or absence of specific cell layers and disruption of normal tissue architecture. One such class of diseases may include bladder disorders, such as interstitial cystitis.

Approximately one million people in the United States suffer from interstitial cystitis, a chronic painful urinary bladder disorder characterized by thinning or ulceration of the bladder epithelial lining; its etiology is unknown. As described herein, the present invention is directed to a novel glycosylated frizzled-related peptide inhibitor of cell proliferation. This antiproliferative factor (APF) profoundly inhibits bladder cell proliferation via regulation of cell adhesion protein and growth factor production. The structure of APF was deduced using ion trap mass spectrometry, enzymatic digestion, lectin affinity chromatography, and total synthesis, and confirmed by coelution of native and synthetic APF derivatives on microcapillary LC/MS. The standard form of APF was determined to be an acidic, heat stable sialoglycopeptide whose peptide chain has 100% homology to the putative 6th transmembrane domain of frizzled 8. Both synthetic and native APF had identical biological activity in normal bladder epithelial cells and T24 bladder cancer cells. Northern blot analysis indicated binding of a probe containing the sequence for the frizzled 8 segment with mRNA extracted from cells of patients with interstitial cystitis but not controls and antibodies raised against synthetic APF peptide bound to purified native APF in a dose-dependent manner. APF is therefore the first frizzled-related peptide growth inhibitor shown to contain exclusively a transmembrane segment of a frizzled protein, and is a potential biomarker for interstitial cystitis.

The reported results demonstrate that the APF made specifically by bladder epithelial cells explanted from patients with interstitial cystitis is a uniquely modified frizzled 8-related sialoglycopeptide with a peptide structure that bears 100% homology to the sixth transmembrane segment of this G-protein-coupled Wnt ligand receptor (Saitoh *et al.,* 2001). This small secreted frizzled-related peptide is normally expressed in human bladder epithelial cells during embryogenesis, and it is an abnormal variant of a frizzled protein that is produced only by bladder epithelial cells from these patients. However, its specificity for this disorder as well as its identification as a secreted frizzled-related peptide growth inhibitor indicates its role in the pathogenesis of this disease. Identification of APF also allows for the possible development of treatment for interstitial cystitis based on inhibition of APF production or activity, or stimulation of APF breakdown. In addition, exclusive expression of APF in adults by bladder epithelial cells from interstitial cystitis patients may provide a direct noninvasive diagnostic test for this disorder.

To date, two disease states have been noted to be associated with abnormally increased expression of secreted frizzled-related proteins other than APF: overload-induced heart failure, in which mRNA for two such proteins have been shown to be elevated in failing ventricles as compared to control hearts (Schumann *et al.*, 2000), and degenerative retinal disease, in which a secreted frizzled-related protein and its mRNA expression are greatly elevated (Jones *et al.*, 2000). The previously identified secreted frizzled-related proteins contain a cysteine-rich extracellular domain, allowing them to inhibit Wnt signaling by one of two mechanisms: by binding to the Wnt ligand, or by forming nonsignalling dimers with frizzled receptors (Bafico *et al.*, 1999). APF is therefore the first frizzled-related growth inhibitor that bears homology only to a transmembrane portion of a frizzled receptor, suggesting that growth inhibition by frizzled-related proteins may also occur *via* additional mechanisms. APF is also the smallest secreted frizzled-related peptide identified to date, with previously described frizzled-related peptides having molecular weights of 33.5-39.9 kDa (Jones and Jomacy, 2002).

Microarray analysis previously indicated that APF decreases the expression of several genes including Jun N-terminal kinase in bladder epithelial cells (Keay *et al.,* 2003), suggesting the possibility that APF may interfere with non-canonical Wnt signaling mediated via a frizzled receptor protein (Pandur *et al.,* 2002). However, linkage of the cytoskeleton to its substratum includes binding of the actin-catenin complex to E-cadherin, a protein whose expression has also been shown to be significantly upregulated by APF in bladder epithelial cells (Keay *et al.,* 2003), and which is known to inhibit canonical Wnt signaling in both urothelial carcinoma and normal urothelial cells *in vitro* (Thievessen *et al.,* 2003). Not to be bound to any theory, thus, in specific embodiments APF mediates its antiproliferative effects *via* inhibition of canonical or non-canonical Wnt pathway(s) in either bladder carcinoma or normal bladder cells, for example.

Although other potent sialylated small glycopeptide growth inhibitors have been isolated from serum or brain of normal mammals and shown to inhibit proliferation of both normal and malignant cells from a variety of tissues (Auger *et al.*, 1989; Moos *et al.*, 1995), no structural data are available for any of these natural growth inhibitors and their relationship to frizzled-related proteins or any specific signaling pathway(s) has not been determined. APF is therefore the first of this class of growth inhibitors to be completely characterized as well as the first to be synthesized. The requirement of the α-O-linked N-acetylhexosamine structure for antiproliferative activity and regulation of growth factor production by APF suggests that APF may bind to a specific cellular receptor(s), as suggested for another sialoglycopeptide growth inhibitor (Sharifi and Johnson, 1987). The extremely hydrophobic nature of the peptide moiety of APF further suggests the possibility that it may interact with the plasma membrane and expose a distinct conformation of the sugar moiety at the cell surface.

### III. Antiproliferative Factor (APF)

The present invention relates to antiproliferative factor (APF). APF comprises a glycopeptide that inhibits proliferation of bladder epithelial cells, skin fibroblasts, and other epithelial cells including prostate cells, and may be generated by bladder epithelial cells, such as those associated with interstitial cystitis. APF may be present in the urine of individuals having interstitial cystitis, and may be generated or biosynthesized by tissues and cells other than urinary bladder tissue and cells. The compound may be considered a toxin, a negative growth factor, or both.

APF was identified because of its ability to inhibit the growth of cells that line the bladder wall, by altering the production of several proteins by these cells, such as specific growth factors and cell adhesion proteins. Not to be bound to any theory, APF may cause interstitial cystitis in which the bladder lining is generally thin and/or ulcerated.

Thus, as used herein the term "APF" refers to a class of compounds wherein the structure in FIG. 4 is merely the prototypical APF and other related compositions are encompassed, so long as they are diagnostic for a bladder condition and/or are useful as a direct or indirect target to treat a disorder associated with abnormally increased cell proliferation, and their inhibitors are useful for treating disorders associated with decreased cell proliferation.

Although in particular aspects of the invention APF comprises the structure provided in FIG. 4, this is merely one embodiment of the invention. A skilled artisan recognizes that the structure in FIG. 4 may be secreted by bladder epithelial cells and is therefore diagnostic of a bladder disorder such as interstitial cystitis. However, in some embodiments a similar but non-identical structure of APF is diagnostic of a bladder disorder. APF compositions herein encompass synthetic APF. Furthermore, the compounds of the present invention may be made using synthetic means.

Furthermore, inhibition of a molecule having the structure of APF in FIG. 4 is useful, but inhibition of a molecule having a similar structure of APF may also be useful. The inhibition may be directed to inhibiting the function of APF, such as with an antibody, for example; or for inhibiting the production of APF, such as an anti-sense oligonucleotide or small interfering RNA, for example; or for stimulating the breakdown of APF; or a combination thereof.

Thus, in specific embodiments, compositions related to the present invention consist of one to six sugar residues; and a peptide, wherein the peptide-linked to one of the sugar moieties at a linking amino acid, wherein the linking amino acid comprises a heteroatom which serves as the linking portion of the linking amino acid. More specifically, the linking amino acid comprises a serine, a threonine, or a cysteine. In other specific embodiments, the compositions of the present invention comprises two or three sugar residues and nine amino acids and the linking amino acid is a threonine or serine.

An APF composition of the invention comprises a peptide, of SEQ ID NO:1, SEQ ID NO:3, or SEQ ID NO:4, SEQ ID NO:5 or SEQ ID NO:6. The peptide comprises at least part of a transmembrane domain, particularly part of frizzled 8, such as a transmembrane domain of frizzled 8. The peptide is hydrophobic.

The glycoprotein comprising a galactose covalently linked to an N-acetylglucosamine or an N-acetylgalactosamine covalently linked to a peptide of SEQ ID NO:1 or variants thereof is provided herein. The term "variants thereof' includes peptidomimetics of various types (Ahn *et al.*, 2002). The peptides may comprise any suitable amino acids, such as L-amino acids, D-amino acids, N-methylated amino acids, or a combination thereof, as well as peptidomimetic compounds such as unnatural amino acids or other "peptide-like" organic constructs that mimic the specific structural elements of a linear, cyclic, or branched peptide that correspond to active peptides. The sugar moieties may be natural, synthetic, carbohydratemimetic, or a mixture thereof may be used in a composition. Glycopeptidomimetic compounds where the sugars are carbohydratemimetic moieties or the peptide components are peptidomimetic moieties, or a combination of the two, are encompassed in the invention. In specific embodiments, the sugars of the present invention include amino sugars.

In a particular aspect of the invention, the APF has a molecular mass of 1482.8 and comprises nine amino acids and three sugar moieties in the following order: (a) Sialic acid-galactose-N-acetylgalactosamine-threonine-valine-proline-alanine-alanine-valine-valine-valine-alanine; or (b) Sialic acid-galactose-Nacetylglucosamine-threonine-valine-proline-alanine-alanine-valine-valine-valine-alanine; or (c) Sialic acid-galactose-N-acetylglucosamine-serine-leucine-proline-alanine-alanine-valine-valine-valine-alanine. The composition may be further defined as having one or more of the following: the sialic acid in (a) is linked to galactose *via* a 2,3 linkage; the sialic acid in (b) is linked to galactose *via* a 2,3 linkage; the sialic acid in (c) is linked to galactose *via* a 2,3 linkage; the galactose in (a) is linked to the N-acetylgalactosamine *via* a 1,3 linkage; the galactose in (b) is linked to the N-acetylglucosamine *via* a 1,4 linkage; the galactose in (c) is linked to the N-acetylglucosamine *via* a 1,4 linkage; the N-acetylglucosamine is linked to serine *via* an O linkage in an alpha configuration; or the N-acetylgalactosamine is linked to threonine or serine *via* an O linkage in an alpha configuration.

It is contemplated that the compounds of the present invention may be modified so as to improve certain characteristics, such as solubility by adding a water soluble unit. The term "water soluble unit" means any functional group imparting water solubility, including, but not limited to, SO₃-, PO₃²⁻, CH₂ COO⁻, a quaternary ammonium group attached *via* an ester or alkyl linkage such as C=O(CH₂)ₓ NAlk₃ or (CH₂)ₓ NAlk₃ where Alk₃ represents three alkyl groups that are independently C1-C4 alkyl and x is 1-4, (CH₂ CH₂ O)ₙ CH₂ CH₂ OX (n=1-3) wherein X may be H or CH₃, *i.e.*, PEG or MeO-PEG. The counterion for water soluble units bearing a charge include, but are not limited to, metals such as alkali and alkaline earth metals, and halogens.

Certain compounds of the present invention comprise a threonine, a serine, or a cysteine at the N- terminus or any functional equivalent. Non-limiting examples of functional equivalents include a synthetic derivative having a primary or secondary or tertiary alcohol, an ester, a carboxylic acid, an ether, a thiol, a thiolate, or any functional group enabling for covalent linkage with a sugar molecule, provided the molecule retains biological function.

Other functionalities contemplated in "derivatives" of the present invention include isomers of any of the sugars or amino acids, whether positional, structural, or stereoisomers. Other substituents known to those skilled in the chemical arts may be provided, so long as the biological function of the molecule is retained.

### IV. Diagnosis

APF compounds of the present invention may be utilized to diagnose one or more bladder disorders.

Identification of an APF compound for an individual indicates an increased risk for developing a particular bladder disorder, and in some cases the symptoms of the disease may not yet be apparent. The identification of a predisposition to developing a bladder condition, such as interstitial cystitis, and the identification of the presence of the disease may use the identification of APF.

The diagnosis of a bladder disorder may comprise detection of APF glycopeptide or its activity, and the detection may be direct detection of the APF molecule or indirect detection, such as of an intermediate, a by-product, a breakdown product, a derivative, and so forth. The detection of APF may employ any suitable means in the art, such as an antibody to the peptide moiety of APF; techniques such as mass spectrometry, nuclear magnetic resonance, or proteomic methods to detect the peptide, sugar, or glycopeptide moiety of APF; or assays for inhibition of normal or malignant cell metabolism or proliferation to detect APF activity, for example. In general, assays used to detect the peptide moiety of APF in a sample derived from an individual are well-known to those of skill in the art and include western blot analysis, ELISA assays, "sandwich" assays, radioimmunoassay, immunohistochemistry or other antibody-based assays (see, *e.g.*, Coligan *et al.*, Current Protocols in Immunology 1(2), Chapter 6, (1991)). Generally, Western blotting is a technique for blotting proteins or peptides onto nictrocellulose, nylon or other transfer membrane after they have been resolved by gel electrophoresis. The proteins can be detected by one of several methods, including autoradiography (if labeled), or through binding to labeled, ¹²⁵I-labeled or enzyme-linked antibodies, lectin or other specific binding agents, for example.

An ELISA assay initially comprises preparing an antibody specific to the antigen, wherein the antigen comprises the peptide moiety and this is preferably a monoclonal antibody. Next, a reporter antibody typically is prepared against the monoclonal antibody. To the reporter antibody is attached a detectable reagent such as a radioactive moiety, fluorescent moiety or enzyme, such as horseradish peroxidase or alkaline phosphatase. A sample is removed from the host and incubated on a solid support, *e.g.*, a polystyrene dish, binding the proteins in the sample. Any free protein binding sites on the dish are then blocked by incubating with a non-specific protein, such as bovine serum albumen or milk proteins. Next, the antibody is incubated in the dish during which time the antibodies attach to any peptide moieties, the sugar moieties, or the glycopeptide moieties of APF from the sample attached to the polystyrene dish. All unbound antibody is washed out with buffer. The reporter antibody is then placed in the dish resulting in binding of the reporter antibody to any monoclonal antibody bound to the peptide moiety of APF. Unattached reporter antibody is then washed out, and the peptide moiety of APF is then detected. Alternatively, the anti-APF antibody may be directly labeled, as above. In specific embodiments, the amount of APF present in a given volume of a patient sample is proportional to the detection of the peptide moiety, the sugar moiety, or the glycopeptide thereof and is compared against a standard curve.

A "sandwich" assay is similar to an ELISA assay. In a "sandwich" assay, the peptide moiety of APF is passed over a solid support and allowed to bind to antibody attached to the solid support. A second antibody is then allowed to bind to the peptide moiety of APF. A third antibody specific to the second antibody is then passed over the solid support and binds to the second antibody, which is thereby detected. Alternatively, the second antibody may be directly labeled, as above.

### V. Therapeutic Embodiments

A skilled artisan recognizes that the APF compositions of the present invention may be addressed in a variety of ways to provide therapy for a bladder condition. In therapeutic embodiments wherein proliferation is undesirable, an APF composition may be employed for therapy.

A deleterious bladder condition may be associated either indirectly or directly with reduced cellular proliferation, such as inhibited epithelial cellular proliferation. Such a condition may result in harmful alterations to the bladder epithelium such that it would be beneficial to reduce or substantially remove the inhibition of cellular proliferation. In such cases, it may be desirable to deliver to the individual with the bladder condition, such as deliver systemically to the individual or directly to bladder epithelium, an inhibitor of an APF composition. Specific examples of APF inhibitors include antibodies to the peptide moieties of the APF composition, as well as oligonucleotides, small interfering RNAs, small compounds that block the interaction of APF with its target, and compounds that cause or stimulate the breakdown of APF, and/or a mixture thereof. The composition may be delivered by any suitable means, although in specific embodiments it is delivered via catheter, orally, intravenously, topically, subcutaneously, transcutaneously, intramuscularly, intra-jointly, parenterally, peritoneally, intranasally, intravesically or by inhalation. The composition may be comprised in a pharmaceutically acceptable excipient, such as an aqueous or non-aqueous liquid. It may be administered in a non-aqueous excipient due to the hydrophobic nature of the peptide moiety. It may be delivered alone or in a carrier, such as a liposome, encapsulated cell, viral vector, nanoparticles, biodegradable gel or polymer, implanted osmotic pump, or other suitable devices.

In another aspect of the invention, a deleterious bladder condition is associated either indirectly or directly with increased cellular proliferation, such as increased epithelial cellular proliferation. Such a condition may result in malignancy of the bladder epithelium, such that it would be beneficial to reduce in part or substantially in full the amount of cellular proliferation. In these aspects, it is desirable to deliver to the individual with the bladder condition, such as deliver an APF composition systemically to the individual or directly to bladder epithelium, an APF composition. The composition may be delivered by any suitable means, although in specific embodiments it is delivered *via* catheter, orally, intravenously, topically, subcutaneously, transcutaneously, intramuscularly, orally, intra-jointly, parenterally, peritoneally, intranasally, intravesically or by inhalation. In other specific embodiments, the composition is comprised in a pharmaceutically acceptable excipient. It may be delivered alone or in a carrier, such as a liposome, encapsulated cell, viral vector, nanoparticles, biodegradable gel or polymer, implanted osmotic pump, or other suitable devices.

In a particular embodiment, an APF composition of the present invention may be administered to an individual with any kind of cancer, including epithelial cancers. In specific embodiments, there is a malignancy of the bladder epithelium, which may be referred to herein as bladder cancer. In specific embodiments, there is a cancer therapy additional to the APF treatment, such as gene therapy, chemotherapy, radiation, surgery, immunotherapy, or a combination thereof.

### VI. Bladder Disorders

Although the present invention may be useful for any medical condition for which APF provides therapy, the present invention may be useful for one or more bladder disorders. Although the term "bladder disorder" refers to any abnormal condition of the urinary bladder, in specific embodiments the bladder disorder comprises interstitial cystitis, bladder cancer, either as a primary or secondary cancer, chronic pelvic pain syndrome, irritable bladder syndrome, urethral syndrome, painful bladder syndrome, chronic nonbacterial prostatitis, and other bladder conditions, for example.

Typical symptoms of interstitial cystitis include pain, which can be in the abdominal, urethral or vaginal area and is also frequently associated with sexual intercourse; urgency, which includes the sensation of having to urinate immediately and may also be accompanied by pressure and/or spasms; and increased frequency of urination, which can be day and/or night frequency of urination.

Diagnosis of intersitial cystitis is heretofore performed using cystoscopy, and hydro-distention and biopsies are normally performed at the same time. Examination by cytoscopy of a typical bladder having interstitial cystitis may identify submucosal pinpoint hemorrhages (glomerulations), thinning of the epithelium and/or Hunner's ulcers; in some cases, inflammation may also be present. Thus, there is considerable pain when urine enters into the bladder of an IC patient, making it very difficult for patients with interstitial cystitis to be able to hold urine in their bladder, due to the burning, stinging and pain.

Current therapies include oral medications, such as Elmiron, Amitriptyline (Elavil) Atarax, Neurontin, Ditropan, Prozac, Cimetidine. Therapeutic agents associated with the present invention may be used either alone or in conjunction with one or more of these or similar medications. The patients may also suffer with various other syndromes including fibromyalgia, urethral syndrome, vulvodynia, irritable bowel syndrome, chronic fatigue syndrome, allergies, and other autoimmune disorders, such as scleroderma.

### VII. Pharmaceutical Compositions

The present invention is also directed to pharmaceutical compositions for use in treating or ameliorating bladder conditions, such as bladder cancer, epithelial hyperplasia or malignancies of epithelial origin, of fibroblast hyperplasia or malignancy, other solid tumors, or lymphoreticular malignancies. The compounds of the present invention are also contemplated for use as an adjuvant treatment for bladder cancer or other malignancies, as an antiangiogenic agent or an antifungal agent. The compounds of the present invention may be used to generate antibodies, antisense oligonucleotides, small interfering RNA, or agents that block the interaction of APF with its target for the treatment of interstitial cystitis or other disorders related to cell proliferation.

Such methods generally involve administering a pharmaceutical composition comprising an effective amount of the glycopeptides of the present invention. Other exemplary compositions include an effective amount of an oligonucleotide encoding the nonapeptide of a sequence as essentially set forth in SEQ ID NO:1, SEQ ID NO:3, SEQ ID NO:4, SEQ ID NO:5.

Where the invention is directed to treating with the compounds of the present invention, administration of the compounds of the invention with a suitable pharmaceutical excipient as necessary can be carried out *via* any of the accepted modes of administration. The compounds may be comprised in a pharmaceutically acceptable excipient, which may be considered as a molecular entity and/or composition that does not produce an adverse, allergic and/or other untoward reaction when administered to an animal, as appropriate. It includes any and/or all solvents, dispersion media, coatings, antibacterial and/or antifungal agents, isotonic and/or absorption delaying agents and/or the like. The use of such media and/or agents for pharmaceutical active substances is well known in the art. Except insofar as any conventional media and/or agent is incompatible with the active ingredient, its use in the therapeutic compositions is contemplated.

Thus, administration can be, for example, intravenous, topical, subcutaneous, transcutaneous, intramuscular, oral, intra-joint, parenteral, peritoneal, intranasal, intravesical or by inhalation. Suitable sites of administration thus include, but are not limited to, skin, bronchial, gastrointestinal, anal, vaginal, eye, bladder, and ear. The formulations may take the form of solid, semi-solid, lyophilized powder, or liquid dosage forms, such as, for example, tablets, pills, capsules; powders, solutions, suspensions, emulsions, suppositories, retention enemas, creams, ointments, lotions, aerosols or the like, preferably in unit dosage forms suitable for simple administration of precise dosages.

The compositions typically include a conventional pharmaceutical carrier or excipient and may additionally include other medicinal agents, carriers, adjuvants, and the like. Preferably, the composition will be about 5% to 75% by weight of a compound or compounds of the invention, with the remainder consisting of suitable pharmaceutical excipients. Appropriate excipients can be tailored to the particular composition and route of administration by methods well known in the art, *e.g.*, REMINGTON'S PHARMACEUTICAL SCIENCES, 18TH ED., Mack Publishing Co., Easton, Pa. (1990).

For oral administration, such excipients include pharmaceutical grades of mannitol, lactose, starch, magnesium stearate, sodium saccharine, talcum, cellulose, glucose, gelatin, sucrose, magnesium carbonate, and the like. The composition may take the form of a solution, suspension, tablet, pill, capsule, powder, sustained-release formulation, and the like.

In some embodiments, the pharmaceutical compositions take the form of a pill, tablet or capsule, and thus, the composition can contain, along with the biologically active conjugate, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof.

The active compounds of the formulas may be formulated into a suppository comprising, for example, about 0.5% to about 50% of a compound of the invention, disposed in a polyethylene glycol (PEG) carrier (*e.g.*, PEG 1000 [96%] and PEG 4000 [4%]).

Liquid compositions can be prepared by dissolving or dispersing compound (about 0.5% to about 20%), and optional pharmaceutical adjuvants in a carrier, such as, for example, aqueous saline (*e.g.*, 0.9% w/v sodium chloride), aqueous dextrose, glycerol, ethanol and the like, to form a solution or suspension, *e.g.*, for intravenous administration. The active compounds may also be formulated into a retention enema.

If desired, the composition to be administered may also contain minor amounts of non-toxic auxiliary substances such as wetting or emulsifying agents, pH buffering agents, such as, for example, sodium acetate, sorbitan monolaurate, or triethanolamine oleate.

For topical administration, the composition is administered in any suitable format, such as a lotion or a transdermal patch. For delivery by inhalation, the composition can be delivered as a dry powder (*e.g.*, Inhale Therapeutics) or in liquid form *via* a nebulizer.

Methods for preparing such dosage forms are known or will be apparent to those skilled in the art; for example, see Remington's Pharmaceutical Sciences, supra., and similar publications. The composition to be administered will, in any event, contain a quantity of the pro-drug and/or active compound(s) in a pharmaceutically effective amount for relief of the condition being treated when administered in accordance with the teachings of this invention.

Generally, the compounds of the invention are administered in a therapeutically effective amount, *i.e.*, a dosage sufficient to effect treatment, which will vary depending on the individual and condition being treated. Typically, a therapeutically effective daily dose is from 0.1 to 100 mg/kg of body weight per day of drug. Most conditions respond to administration of a total dosage of between about 1 and about 30 mg/kg of body weight per day, or between about 70 mg and 2100 mg per day for a 70 kg person. However, it is possible that an effective dose of APF, especially if administered directly into the bladder, may be outside of this range.

Stability of the conjugate can be further controlled by chemical alterations, including D amino acid residues in the polypeptide chain as well as other peptidomimetic moieties. Furthermore, stability of the conjugates could also be enhanced by unnatural carbohydrate residues.

### VIII. Combination Treatments

In order to increase the effectiveness of an APF composition for the treatment of cancer in an individual, such as a patient, it may be desirable to combine these compositions with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy, for example, by combining it with other cancer therapies. In the context of the present invention, it is contemplated that APF composition therapy could be used similarly in conjunction with chemotherapeutic, radiotherapeutic, surgical, or immunotherapeutic intervention, in addition to other pro-apoptotic or cell cycle regulating agents.

Alternatively, the APF treatment may precede, follow, or both the other agent treatment by intervals ranging from minutes to weeks. In embodiments where the APF composition and the other agent are applied separately to a cell of the individual, one would generally ensure that a significant period of time did not expire between the time of each delivery, such that the APF composition and the other agent would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

Various combinations may be employed, for example, wherein the APF treatment is "A" and the secondary agent, such as radio- or chemotherapy, is "B":
A/B/A B/AB B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the APF compositions of the present invention to a patient will follow general protocols for the administration of chemotherapeutics, taking into account the toxicity, if any, of the molecule. It is expected that the treatment cycles would be repeated as necessary. It also is contemplated that various standard therapies, as well as surgical intervention, may be applied in combination with the described hyperproliferative cell therapy.

### A. Chemotherapy

A skilled artisan recognizes that in addition to the APF treatment described herein for the purpose of inhibiting cell growth, other chemotherapeutic agents are useful in the treatment of neoplastic disease. Examples of such chemotherapeutic agents include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, famesyl-protein tansferase inhibitors, transplatinum, 5-fluorouracil, vincristin, vinblastin and methotrexate, or any analog or derivative variant of the foregoing.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, etc.) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy, thus, could be used as part of a combined therapy, in conjunction with APF therapy. The general approach for combined therapy is discussed below. Generally, the tumor cell must bear some marker that is amenable to targeting, *i.e.*, is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155.

### D. Genes

The secondary treatment may be a secondary gene therapy in which a second therapeutic polynucleotide is administered before, after, or at the same time as an APF molecule, having a combined anti-hyperproliferative effect on target tissues. A variety of proteins are encompassed within the invention, including inhibitors of cellular proliferation, such as tumor suppressors, including p53; and/or regulators of programmed cell death, such as Bcl-2.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). The present invention may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other agents

Other agents may be used in combination with the present invention to improve the therapeutic efficacy of treatment. These additional agents include immunomodulatory agents, agents that affect the upregulation of cell surface receptors and GAP junctions, cytostatic and differentiation agents, inhibitors of cell adehesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abililties of the present invention by establishment of an autocrine or paracrine effect on hyperproliferative cells. Increases intercellular signaling by elevating the number of GAP junctions would increase the anti-hyperproliferative effects on the neighboring hyperproliferative cell population. Cytostatic or differentiation agents can be used in combination with the present invention to improve the anti-hyerproliferative efficacy of the treatments. Inhibitors of cell adehesion may improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

Hormonal therapy may also be used in conjunction with the present invention or in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

### IX. Kits

Kits associated with the glycoproteins of the present invention comprise another aspect of the present invention. Such kits will generally contain, in suitable container means, an APF molecule of the present invention, namely a glycoprotein comprising a peptide having a sequence as set forth in SEQ ID NO:1, SEQ ID NO:3, SEQ ID NO:4, SEQ ID NO:5 or SEQ ID NO:6 or a means to detect such as molecule. The kit may have a single container means that contains the APF composition or it may have distinct container means for the APF composition and other reagents that may be included within such kits.

The components of the kit may be provided as liquid solution(s), or as dried powder(s). When the components are provided in a liquid solution, the liquid solution is an aqueous or non-aqueous solution, with a sterile aqueous or non-aqueous solution being particularly preferred. When reagents or components are provided as a dry powder, the powder can be reconstituted by the addition of a suitable solvent. It is envisioned that the solvent may also be provided in another container means.

Disclosed herein are Kits comprising reagents for detecting peptides having a sequence as set forth in SEQ ID NO:1, SEQ ID NO:3, SEQ ID NO:4, or SEQ ID NO:5, such as is required for immunoassay. The immunodetection reagent will typically comprise a label associated with the antibody or antigen, or associated with a secondary binding ligand. Exemplary ligands might include a secondary antibody directed against the first antibody or antigen or a biotin or avidin (or streptavidin) ligand having an associated label. Of course, a number of exemplary labels are known in the art and all such labels may be employed. The kits may contain antibody-label conjugates either in fully conjugated form, in the form of intermediates, or as separate moieties to be conjugated by the user of the kit.

The container means will generally include at least one vial, test tube, flask, bottle, syringe or other container means, into which the antigen or antibody may be placed, and preferably suitably aliquoted. Where a second binding ligand is provided, the kit will also generally contain a second vial or other container into which this ligand or antibody may be placed. The kits of the present invention will also typically include a means for containing the containers in close confinement for commercial sale. Such containers may include injection or blow-molded plastic containers into which the desired vials are retained.

### X. APF Antibodies

Also described herein is an antibody that is immunoreactive with a proteinaceous, such as a peptide of the invention. The peptide may be comprised as part of a larger composition, such as an APF of the present invention.

An antibody can be a polyclonal or a monoclonal antibody. Means for preparing and characterizing antibodies are well known in the art (See, *e.g*., Howell and Lane, 1988).

Briefly, a polyclonal antibody is prepared by immunizing an animal with an immunogen comprising a peptide, sugar or glycopeptide of the present invention, with or without an adjuvant or other such molecules that enhance immune responses (*e.g*. KLH) and collecting antisera from that immunized animal. A wide range of animal species can be used for the production of antisera. Typically an animal used for production of anti-antisera is a rabbit, a mouse, a rat, a chicken, a hamster or a guinea pig. Because of the relatively large blood volume of rabbits, a rabbit is a preferred choice for production of polyclonal antibodies.

Antibodies, both polyclonal and monoclonal, specific for APF peptides, represented by SEQ ID NO:1, SEQ ID NO:3, SEQ ID NO:4, or SEQ ID NO:5, variants and epitopes thereof, may be prepared using conventional immunization techniques, as will be generally known to those of skill in the art. A composition containing antigenic epitopes of APF may be used to immunize one or more experimental animals, such as a rabbit, chicken, or mouse, which will then proceed to produce specific antibodies against the peptide, sugar, or glycopeptide regions of APF. Polyclonal antisera may be obtained, after allowing time for antibody generation, simply by bleeding the animal and preparing serum samples from the whole blood.

To obtain monoclonal antibodies, one would also initially immunize an experimental animal, often preferably a mouse, with an APF composition. One would then, after a period of time sufficient to allow antibody generation, obtain a population of spleen or lymph cells from the animal. The spleen or lymph cells can then be fused with cell lines, such as human or mouse myeloma strains, to produce antibody-secreting hybridomas. These hybridomas may be isolated to obtain individual clones which can then be screened for production of antibody to the desired APF peptide.

Following immunization, spleen cells are removed and fused, using a standard fusion protocol with plasmacytoma cells to produce hybridomas secreting monoclonal antibodies against APF and/or APF peptides. Hybridomas which produce monoclonal antibodies to the selected antigens are identified using standard techniques, such as ELISA and Western blot methods. Hybridoma clones can then be cultured in liquid media and the culture supernatants purified to provide the APF-specific monoclonal antibodies.

It is proposed that the monoclonal antibodies here described will find useful application in standard immunochemical procedures, such as ELISA and Western blot methods, as well as other procedures which may utilize antibody specific to APF epitopes.

Additionally, it is proposed that monoclonal antibodies specific to the particular peptide may be utilized in other useful applications. For example their use in immunoabsorbent protocols may be useful in purifying native or recombinant APF species or variants thereof.

In general, both poly- and monoclonal antibodies against APF may be used. For example, they may be employed in antibody cloning protocols to obtain cDNAs or genes encoding APF or related proteins. This may also be used to select for hybridoma cells or lymphocytes that recognize the APF epitopes or make antibodies or segments thereof that recognize APF epitopes. They may also be used in inhibition studies to analyze the effects of APF in cells or animals. Anti-APF antibodies will also be useful in immunolocalization studies to analyze the distribution of APF peptides during various cellular events, for example, to determine the cellular or tissue-specific distribution of the APF peptide or glycopeptide under different physiological conditions. A particularly useful application of such antibodies is in purifying native or recombinant APF peptides or glycopeptides, for example, using an antibody affinity column. The operation of all such immunological techniques will be known to those of skill in the art in light of the present disclosure.

Also disclosed are methods of generating antibodies to an APF composition, such as to a peptide moiety. The peptide moities are selected from SEQ ID NO:1, SEQ ID NO:3, SEQ ID NO:4, SEQ ID NO:5, or SEQ ID NO:6. These antibodies may then be utilized for detection of a bladder condition, such as interstitial cystitis, for example; for diagnosis of a bladder condition; or for therapeutic purpose, such as to inhibit an APF composition.

### EXAMPLE 1

### EXEMPLARY METHODS AND REAGENTS

### Cell Cultures (For Reference)

Cystoscopy was performed under general anesthesia, and 4 mm2 pieces of transitional epithelium with submucosa bladder tissue were obtained using rigid cold cup biopsy forceps from six patients who had previously undergone cystoscopy and fulfilled the NIDDK/NIH diagnostic criteria for interstitial cystitis, and six age- race- and gender-matched controls who were asymptomatic for urinary tract disease, as previously described (Keay *et al.,* 2001; *Keay et al.,* 2000; *Keay et al.,* 2003a; *Keay et al.,* 2003b; *Keay et al.,* 1996). All patients were at least 18 years old and enrolled in accordance with guidelines of the Institutional Review Board of the University of Maryland School of Medicine.

Explanted epithelial cells were propagated from these biopsy specimens in DMEM-F12 (Media Tech, Herndon, VA) with 10% heat inactivated fetal bovine serum (FBS), 1% antibiotic/antimycotic solution, 1% L-glutamine, 1.0 U/ml insulin (all from Sigma, St. Louis, MO), and 5 µg/ml hEGF (R & D Systems, Minneapolis, MN) at 370 C in a 5% CO2 atmosphere, and characterized by binding of AE-1/AE-3 pancytokeratin antibodies (Signet, Dedham, MA), as previously described (Keay *et al.,* 2001; *Keay et al.,* 2000; Keay *et al.,* 2003a; Keay *et al.,* 2003b; Keay *et al.,* 1996).

T24 bladder carcinoma cells (ATCC #HTB 4) were purchased from the American Type Culture Collection (Rockville, MD) and cultured in McCoy's 5A medium (Gibco-Invitrogen, Carlsbad, CA) containing 10% FBS, 1% glutamine and 1% antibiotic/antimycotic solution (all from Sigma).

### APF Purification (For Reference)

APF was harvested from the supernatant of explanted patient bladder epithelial cells and purified using molecular weight fractionation, ion exchange chromatography, hydrophobic interaction chromatography, and reversed phase high performance liquid chromatography (HPLC), as previously described (Keay *et al*., 2000). Mock APF was prepared using the supernatant of normal control bladder epithelial cells and the same purification procedure.

### Microcapillary LC-MS/MS Analysis (For Reference)

Microcapillary reversed-phase liquid chromatography (mRPLC) was performed using an Agilent 1100 capillary LC system (Agilent Technologies, Palo Alto, CA) coupled online to an ion trap mass spectrometer (LCQ Deca XP, ThermoFinnigan, San Jose, CA). Reversed-phase separations of each sample were performed using 75µm i.d. x 10 cm long fused silica electrospray ionization capillary columns (Polymicro Technologies, Phoenix, AZ) that were slurry packed in house with 3 µm, 300 A pore size C-18 stationary phase (Vydac Hesperia, CA). After sample injection, the column was washed for 20 min with 98% solvent A (0.1 % v/v formic acid in water) and gradient elution was conducted using a linear step gradient from 2% solvent B (0.1% v/v formic acid in acetonitrile) to 42% solvent B in 40 minutes, then from 42% to 95% B in 15 min, at a constant flow rate of 0.5 µL/min.

The ion trap mass spectrometer was operated in a data dependent mode in which each full mass spectrometry (MS) scan was followed by three tandem MS scans where the three most abundant molecular ions were dynamically selected for collision-induced dissociation (CID) using a normalized collision energy of 38%. The temperature of the heated capillary and electrospray voltage were 180 °C and 1.8 kV, respectively.

### ³H-Thymidine Incorporation (For Reference)

Cell proliferation was measured by ³H-thymidine incorporation into explanted normal human bladder epithelial cells, as previously described (Keay *et al.,* 2001; Keay *et al.,* 2000; *Keay et al.,* 2003a; *Keay et al.,* 2003b; *Keay et al.,* 1996). Briefly, purified native or synthetic APF (or the appropriate mock preparation) was diluted in serum-free MEM (containing only glutamine and antibiotics/antimycotics) and applied to the cells; cell controls received serum-free MEM alone. Cells were then incubated at 37°C in a 5% CO₂ atmosphere for 48 hours. The cells were then labeled with 1 µCi/well ³H-thymidine for 4 hours, trypsinized, insoluble cell contents harvested and methanol-fixed onto glass fiber filter paper, and the amount of radioactivity incorporated determined. Significant inhibition of 3H-thymidine incorporation was defined as a mean decrease in counts per minute of greater than 2 standard deviations from the mean of control cells for each plate.

### Cell Count Determination (For Reference)

For cell count experiments, explanted normal human bladder epithelial cells, LNCaP prostate carcinoma cells, or T24 bladder carcinoma cells were plated onto Coming 24 well tissue culture plates (VWR Scientific Products) in MEM (primary bladder cells), DMEM (LNCaP cells), or McCoy's 5A medium (T24 cells) containing 10% FBS, 1% antibiotic/antimycotic solution, 1% L-glutamine at a density of 1 x 10⁴ cells/well and incubated at 37°C in a 5% CO₂ atmosphere overnight. On the next day the medium was removed and replaced with serum-free MEM, DMEM, or McCoy's medium containing 1% antibiotic/antimycotic solution and 1% L-glutamine (all three cell types). HPLC-purified or synthetic APF (or their mock preparations) were then added to the medium and cells further incubated at 37°C with 5% CO₂. Forty-eight hours later the culture supernatant was removed for growth factor analysis by ELISA, cells were trypsinized, stained with Trypan blue (Sigma) and counted using a hemacytometer.

### ELISAs (For Reference)

HB-EGF - ELISAs were performed as previously described (Keay *et al.,* 2001; Keay *et al.,* 1998), coating 96 well Immulon II plates (Dynatech Laboratories, Chantilly, VA) with 200 λ culture supernatant at 40°C overnight. Plates were subsequently rinsed, blocked, and anti-HB-EGF antibody (1 µg/ml) (R & D Systems, Minneapolis, MN) applied. Following additional incubation and rinses, biotinylated anti-goat IgG/avidin D horseradish peroxidase was added. Binding was detected by development with ABTS [2,2'-Azino-bis-(3-ethylbenzothiazoline-6-sulfonic acid)]; absorbance was read at 405 nm.

EGF - Culture supernatant (200 λ) was pipetted into wells precoated with monoclonal anti-EGF antibody, according to the manufacturer's instructions (R & D Systems). Following incubation and rinses, HRP-linked polyclonal anti-EGF was added and binding detected by development with tetramethylbenzidine (TMB) substrate; absorbance was read at 450 nm.

Linear absorbance vs. concentration curves were prepared from results with known concentrations of EGF or HB-EGF, and sample concentrations were determined by plotting absorbance values.

### Enzymatic Cleavage (For Reference)

Partially purified APF was incubated with any of three positionally-specific neuraminidases [2-3; 2-3,6; 2-3,6,8,9] (all from Sigma) at 37°C for 2 hours; control APF was incubated under the same conditions but without enzyme. APF was then purified from enzyme by ultrafiltration using a 3000 MW cut-off Centricon filter (Amicon, Bedford, MA), and antiproliferative activity and lectin-binding of each ultrafiltrate determined.

### Lectin Binding (For Reference)

HPLC purified APF or enzymatically-treated APF samples were applied to the following agarose-conjugated lectins and eluted according to the manufacturer's instructions: wheat germ agglutinin, concanavalin A, lentil lectin (all from Amersham), Vicia villosa (Sigma), Erythrina cristagalli, Griffonia simplicifolia I and II, peanut agglutinin, Jacalin lectin (all from Vector Labs, Burlingame, CA), or Tritrichomonas mobilensis lectin (Calbiochem-Novobiochem Corp., San Diego, CA). Eluates were tested for antiproliferative activity by the ³H-thymidine incorporation assay.

### Sucrose Density Gradient Isoelectric Focusing (For Reference)

HPLC-purified APF was fractionated by high-speed electrofocusing in a pH 2 to 10 sucrose density gradient formed at 15 W for 18 hours with an LKB 8100-1 column (LKB Instruments Inc., Gaithersburg, MD). The pH of the fractions was determined at 40°C, and antiproliferative activity of the neutralized (pH 7.0) fractions was determined in normal bladder cells using the ³H-thymidine incorporation assay.

### APF Synthesis

The synthesis of the peptides was carried out by solid phase methods on the Nautilus 2400 synthesizer (Argonaut Technologies, Foster City, CA) utilizing standard Fmoc chemistry on alanyl 2-chlorotrityl resin (Calbiochem-Novobiochem). Fmoc-protected amino acids (Anaspec Inc., San Jose, CA) were coupled utilizing N-{(dimethylamino)-1H-1,2,3-triazolo[4,5-b]pyridin-1-ylmethylene}-N-methylmethanaminium hexafluorophosphate N-oxide (HATU) (Sigma-Aldrich, Milwaukee, WI) and 1-hydroxy-7-azabenzotriazole (HOAt) (Anaspec, Inc.) reagents. All other reagents were purchased from Sigma-Aldrich. All intermediates and the final products were verified by mass spectrometry.

Fmoc protected O-α-(N-acetyllactosamine)-L-Threonine. Fmoc-L-Thr (Calbiochem-Novabiochem) was converted to phenacyl ester and glycosylated with 2-azido-1-α-bromo-hexa-O-acetyl-2-deoxylactose in the presence of silver triflate according to a slight modification of the procedure by Leuck and Kunz (Leuck and Kunz, 1997). The reaction was carried out a -40°C that ensured >98% selectivity for the α-anomer. The anomeric purity was determined by proton NMR spectroscopy. The phenacyl ester was de-protected by zinc/acetic acid/acetic anhydride, which also resulted in the simultaneous reduction of the azido group and acetylation of the resulting amino group (Svarovsky and Barchi, 2003). The final product was purified by preparative, reverse phase (C8 column) HPLC.

Fmoc protected O-β-(N-acetyllactosamine)-L-Threonine. The procedure for production of the β anomer was identical to that for production of the α anomer except that the glycosylation of threonine by 2-azido-1-α-bromo-hexa-O-acetyl-2-deoxylactose was carried out at -20°C. The product generated by this procedure was a mixture of the α (90%) and β (10%) anomers, which were readily separated by silica gel flash chromatography using an ethyl acetate/hexanes gradient.

Fmoc protected O-α-[Galβ(1->3)GalNAc]-L-Threonine. The synthetic procedure was similar to the method used to produce the Fmoc protected O- α-(N-acetyllactosamine)-L-Threonine. Fmoc-L-Threonine phenacyl ester was glycosylated by the trichloroacetimidate-disaccharide donor in the presence of boron trifluoride diethyl etherate, following the procedure published by Qiu *et al.* (Qiu *et al.,* 1996) with slight modifications. The conversion of the azido group and the deprotection of the phenacyl ester were identical to the procedures used in the Fmoc protected O-α-(N-acetyllactosamine)-L-Threonine synthesis.

General method for glycopeptide synthesis. The glycosylated Fmoc-protected threonine was activated by HATU/HOAt and added to the growing peptide chain in presence of Hunig's base for a prolonged coupling time (16 hours). The glycopeptide was cleaved from the resin with a mixture of trifluoroacetic acid, water, tri-isopropylsilane (90:5:5 v/v/v), the solvent was removed in vacuo, and the residue was dried under high vacuum. The crude, dry glycopeptide was dissolved in anhydrous methanol and treated with sodium methoxide powder for 30 min. When HPLC-MS indicated the complete removal of the acetyl groups, the reaction was stopped with acetic acid and evaporated to dryness. The crude deacetylated product was purified by preparative HPLC using a C8 reverse phase column.

Sialylation of N-terminal threonine hexosamide residue. The N-acetylhexosamine derivatives of the peptides were sialylated enzymatically using recombinant rat α-2,3 (N) sialyltransferase (EMD Biosciences, Inc., La Jolla, CA) and CMP-N-acetyl neuraminic acid substrate (Sigma) in 250 mM MOPS buffer pH 7.4. All crude glycopeptides were purified by reverse phase HPLC on a C8 column, and the purified peptides were analyzed by mass spectrometry.

### Anti-APF Antibodies (For Reference)

Synthetic APF peptide was generated by preparing an APF composition comprising a peptide moiety comprised of a derivative of SEQ ID NO:1, in which an N-Terminal cysteine residue was added (SEQ ID NO:6). The peptide was coupled to KLH at the cysteine residue (serving as the linking amino acid) *via* the sulfur atom, and injected (0.5 mg peptide/rabbit) into two New Zealand White rabbits for antibody production. Following two monthly booster injections, serum was harvested, immunoglobulin fraction purified, and its reactivity against HPLC-purified native APF and KLH tested in a dot-blot format, using HRP-conjugated goat anti-rabbit IgG for the secondary antibody. As shown in FIG. 7, these antibodies detected APF and KLH, and a standard curve against known varying concentrations of APF could be generated. Preimmune antibodies did not bind to either APF or KLH.

### EXAMPLE 2 (For Reference)

### IDENTIFICATION OF AMINO ACIDS BY MASS SPECTROMETRIC ANALYSIS

An APF molecule may be identified and/or characterized by any suitable means in the art, such as through the characterization of the peptide, sugar, or glycopeptide moiety of APF. Mass spectrometry may be utilized. Techniques such as nuclear magnetic resonance or proteomic techniques (including isotope-coded affinity assays), and sensitive chromatographic methods may be utilized, for example.

Microcapillary reversed-phase liquid chromatography was used to obtain extremely pure preparations of APF peptide for mass spectrometry. Analysis of three preparations of HPLC-purified APF using the microcapillary technique indicated the presence of three peptide peaks in approximately equal proportions in each preparation, only one of which had antiproliferative activity against primary bladder epithelial cells *in vitro.* Ion trap mass spectrometric analysis of the active peak indicated a molecular weight of 1482.8 Daltons (FIG. 1A). Analysis of peaks generated by collision-induced dissociation of the active substance indicated a terminal sialic acid linked to a hexose moiety, which in turn was linked to an N-acetylhexose moiety (FIG. 1B); detectable amino acid moieties present from the N terminus following further dissociation of the 827 Dalton peptide moiety included alanine-alanine-valine-valine-valine-alanine (FIG. 1C). The remaining N-terminal amino acids present had a combined molecular weight of 351.4 Daltons. Search for a gene encoding a homologous peptide indicated 100% homology between a sequence with the appropriate total molecular weight (T-V-P-A-A-V-V-V-A: SEQ ID NO:1) and amino acids 541-549 in the 6th transmembrane region of frizzled 8, a Wnt ligand receptor (Qiu *et al.,* 1996).

The peptide moiety may also comprise conservative variants of SEQ ID NO:1, namely a peptide comprising the amino acid sequence of S-V-P-A-A-V-V-V-A (SEQ ID NO:3); T-V-P-A-A-V-V-L-A (SEQ ID NO:4); S-L-P-A-A-V-V-V-A (SEQ ID NO:5); or C-T-V-P-A-A-V-V-V-A (SEQ ID NO:6). It is contemplated that these conservative variants include known conservative substitutions or additions, as in the case of SEQ ID NO:6, defined by chemical properties. For example, serine, threonine and cysteine comprise heteroatoms (atoms other than carbon or hydrogen having nucleophilic or electrophilic properties exploitable as linking means), specifically oxygen (O) and sulfur (S) which provides a means to link to at least one sugar moiety of the present compositions.

The skilled artisan is aware that amino acid substitutions can be made that do not change substantially the functionality of the peptide and/or polypeptide. Therefore, the present invention contemplates a glycopeptide comprising a peptide having a sequence as set forth in SEQ ID NO:1. The term "a sequence essentially as set forth in SEQ ID NO:1" means that the sequence substantially corresponds to a portion of SEQ ID NO:1 and has relatively few amino acids which are not identical to, or a biologically functional equivalent of, the amino acids of SEQ ID NO:1. The term "biologically functional equivalent" is well understood in the art and is further defined in detail herein, as a protein having a sequence essentially as set forth in SEQ ID NO: 1 and that is associated with a diseased state of an epithelial cell. Accordingly, sequences which have 70% and about 80%; or more preferably, between about 85% and about 90%; or even more preferably, between about 90 and 95% and about 99%; of amino acids which are identical or functionally equivalent to the amino acids of SEQ ID NO:1 will be sequences which are "essentially as set forth in SEQ ID NO:1". i.e. SEQ ID NOS: 3, 4, 5 and 6. The APF molecule still comprises activity by substituting more than one amino acid and may still be active.

### Biological Functional Equivalents

Modification and changes may be made in the structure of DNA segments which encode the peptides of the APF of the invention and still obtain a functional molecule that encodes a protein or peptide with desirable characteristics. The following is a discussion based upon changing the amino acids of a protein to create an equivalent, or even an improved, second-generation molecule. The amino acid changes may be achieved by changing the codons of the RNA sequence, according to the following codon table:

**TABLE 1**

| **Amino Acids** | | | **Codons** |
|---|---|---|---|
| Alanine | Ala | A | GCA GCC GCG GCU |
| Cysteine | Cys | C | UGC UGU |
| Aspartic acid | Asp | D | GAC GAU |
| Glutamic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUG CUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGG CGU |
| Serine | Ser | S | AGC AGU UCA UCC UCG UCU |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and messenger RNA sequence, and nevertheless obtain a protein with like properties. It is thus contemplated by the inventors that various changes may be made in the peptide sequences of the disclosed compositions, or corresponding DNA or RNA sequences which encode said peptides without appreciable loss of their biological utility or activity.

In making such changes, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

Each amino acid has been assigned a hydropathic index on the basis of their hydrophobicity and charge characteristics (Kyte and Doolittle, 1982), these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (4.5).

It is known in the art that certain amino acids may be substituted by other amino acids having a similar hydropathic index or score and still result in a protein with similar biological activity, i.e., still obtain a biological functionally equivalent protein. In making such changes, the substitution of amino acids whose hydropathic indices are within .+-.2 is preferred, those which are within .+-.1 are particularly preferred, and those within .+- 0.05 are even more particularly preferred.

It is also understood in the art that the substitution of like amino acids can be made effectively on the basis of hydrophilicity. U.S. Pat. No. 4,554,101 states that the greatest local average hydrophilicity of a protein, as governed by the hydrophilicity of its adjacent amino acids, correlates with a biological property of the protein.

As detailed in U.S. Pat. No. 4,554,101, the following hydrophilicity values have been assigned to amino acid residues: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those which are within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

### EXAMPLE 3 (For Reference)

### IDENTIFICATION OF SUGAR MOIETIES BY LECTIN BINDING ANALYSIS

An APF molecule is identified and/or characterized by any suitable means in the art. Lectin binding analysis may be utilized to identify sugar moieties. Other methods for sugar identification including, but not limited to, chemical degradation, NMR spectroscopy, mass spectrometry, and antibody binding can also be used to identify sugar moieties.

To determine the identity and linkage of the hexose and hexosamine moieties, HPLC-purified APF was incubated in its native state with various agarose-conjugated lectins and the eluates tested for antiproliferative activity (Table 2).

**Table 2: Lectin Binding Analysis**

| **Lectin** | **Reported Specificity** | **APF Binding** | |
|---|---|---|---|
| | | **Native** | **Neuraminidase-treated** |
| Wheat germ agglutinin | sialic acid; terminal GlcNAc | + | ND |
| Tritrichomonas mobilensis | sialic acid | + | ND |
| Peanut agglutinin | Galβ1-3GalNAc; Galactose | - | + |
| Concanavalin A | Mannose, Glucose | - | - |
| Lentil lectin | Mannose, Glucose; terminal GlcNAc | - | - |
| Vicia villosa | Terminal GalNAc; Galα1-3GalNAc | - | - |
| Griffonia simplicifolia I | Galactose; GalNAc | - | + |
| Griffonia simplicifolia II | Terminal GlcNAc | - | - |
| Jacalin | GalNAc; Galactose | - | + |
| Erythrina cristagalli | Galβ1-4GlcNAc; Galactose | - | + |

Native APF bound to wheat germ agglutinin and Tritrichomonas mobilensis lectins, but not to a variety of other lectins, confirming the likely presence of a terminal sialic acid residue. Treatment of native APF with a neuraminidase that cleaves sialic acid linked by any of four known linkages [2,3; 2,6; 2,8; or 2,9] did not decrease its biological activity, but did allow subsequent binding of APF to Griffonia simplicifolia I, peanut agglutinin, Jacalin lectin and Erythrina cristagalli with elution of biologically active toxin. These results indicate the possible presence of galactose and N-acetyllactosamine (galactose β1-4 N-acetylglucosamine), respectively. Apparent lack of binding of the desialylated APF to Vicia villosa lectin indicated that the disaccharide moiety remaining did not consist of galactosα1-3N-acetylgalactosamine, and lack of binding to Griffonia simplicifolia II confirmed that the N-acetylglucosamine moiety was not terminal following removal of sialic acid. The sialic acid was subsequently proven to be linked via a 2,3 bond to the galactose moiety by demonstration of binding to Griffonia simplicifolia I and Erythrina cristagalli lectins after digestion with neuraminidase specific for 2,3 bond cleavage. The deduced structure of APF was therefore sialic acid 2,3 linked to either galactose β1-4 N-acetylglucosamine or galactose β1-3 N-acetylgalactosamine, which was in turn O-linked to threonine on the N-terminus of the peptide. Isoelectric focusing of the complete sialylated native APF indicated an acidic peptide with an isoelectric point of approximately 2.3.

### EXAMPLE 4

### TOTAL SYNTHESIS OF APF

The invention encompasses synthetic APF molecules having antiproliferative activity, particularly for bladder cells, comprising one or more sugar moieties and a hydrophobic moiety. In particular, an APF molecule is synthesized by any suitable means in the art. In specific embodiments, however, the molecule is synthesized as described in this Example.

Because APF bound to Erythrina cristagalli, and because it is a secreted rather than membrane-bound peptide, synthesis started with a N-acetylglucosamine (GlcNAc) linked to threonine using both α and β-glycosylated 'amino acid building blocks for solid phase synthesis of the nonaglycopeptide. A chemoenzymatic approach was taken to construct the complete molecule via synthesis of appropriately Fmoc-protected N-acetyllactosamine-threonine glycoamino acids in both configurations. These were then incorporated into a peptide chain containing the other eight amino acids. Because it was known from the lectin binding studies that the sialic acid moiety was not necessary for APF activity, both α and β -N-acetyllactosamine modified peptides were then assayed for their antiproliferative activity as well as their ability to regulate specific growth factor production by explanted primary normal bladder epithelial cells.

Like native APF, the nonsialylated alpha anomer of the N-acetyllactosamine derivative was a potent inhibitor of cell proliferation, having an IC₅₀ of approximately 0.4 nM; maximal inhibition was possible using as little as 1 nM of the synthetic toxin (FIG. 2). In comparison, the beta anomer of the N-acetyllactosamine derivative and the nonglycosylated peptide had less measurable activity. The neuraminic acid unit was therefore then added to the alpha anomer using recombinant 2,3-sialyltransferase, and the antiproliferative activity of the sialylated derivative determined to be similar to the nonsialylated glycopeptide (FIG. 2). The same 1 nM concentration of sialylated or nonsialylated alpha anomeric synthetic APF that maximally inhibited 3H-thymidine incorporation was also shown to significantly decrease cell counts (data not shown), as well as significantly decrease HB-EGF production and increase EGF production by primary bladder cells (Table 3). APF as determined by all of these analyses was therefore an alpha anomeric sialoglycopeptide.

**Table 3: Effect of Native APF vs. Synthetic APF and its Derivatives on Bladder Epithelial Cell Growth Factor Production**

| | HB-EGF (ng/ml) | EGF (ng/ml) |
|---|---|---|
| Native APF | 0.18 ± 0.25* | 0.49 ± 0.14* |
| Mock APF | 5.24 ± 1.8 | 0.02 ± 0.02 |
| SynthAPF (hexNAc/sial) | 0.13 ± 0.11^{†} | 0.54 ± 0.12^{†} |
| Synth APF (hexNAc) | 0.05 ± 0.1^{†} | 0.38 ± 0.01^{†} |
| Synth APF (peptide alone) | 4.65 ± 0.5 | 0.001 ± 0.002 |

| | | |
|---|---|---|
| *p<0.000 compared to Mock APF ^{†}p<0.0001 compared to Peptide alone | | |

However, while a beta anomeric form of N-acetylglucosamine linked to threonine has been previously described in eucaryotic cells, the alpha anomeric form has not, whereas O-α-N-acetylgalactosamine is commonly found in modified eucaryotic cell proteins. Therefore APF was synthesized to include the alpha anomeric form of N-acetylgalactosamine linked β1->3 to galactose. As also shown in FIG. 2, this synthetic APF had activity similar to the synthetic compound comprising an alpha N-acetylglucosamine. Microcapillary LC-MS/MS in which desialylated native APF was spiked with either the GalNAc-containing or GlcNAc-containing synthetic glycopeptide (FIGS. 3A and 3B) was therefore used to establish the correct structure for APF. Both the native and synthetic GalNAc-containing APF derivatives had an identical retention time that was readily distinguishable from that of the GlcNAc-containing compound. The correct structure of APF is therefore the GalNAc-containing sialoglycopeptide shown in FIG. 4.

### EXAMPLE 5

### INHIBITION OF BLADDER CANCER CELL PROLIFERATION BY NATIVE AND SYNTHETIC APF

Additional evidence that the synthetic and native APF were the same was provided by measurement of their antiproliferative activities against a bladder carcinoma T24 cell line, using live cell count as determined by trypan blue exclusion. As shown in FIG. 5, these cells were sensitive to both native and synthetic APF species at approximately the same concentration.

### EXAMPLE 6

### THERAPEUTIC EMBODIMENTS WITH APF

In some aspects of the invention, it is beneficial to deliver to an individual in need thereof an APF composition to provide its anti-cell proliferation activity. For example, for treating bladder cancer an APF composition is delivered to an individual suffering from at least one symptom or finding (sign) of bladder cancer or to an individual suspected of having bladder cancer. FIG. 8 demonstrates antiproliferative activity against normal human bladder epithelial cells of 2 different peptides (#1, corresponds to SEQ ID NO:1, and #4, corresponds to SEQ ID NO:5) and their sugar derivatives, having two (#2 and #5, each unsialyated) and three (#3 and #6) sugar moieties.

In other embodiments, prostate cancer cells (such as the exemplary LNCaP cells) are treated with APF compositions. LNCaP cells were plated at 2 x 10⁴ cells per well of a 24 well tissue culture plate in DMEM medium containing 10% fetal bovine serum, 1% L-glutamine, 1% antibiotic/antimycotic solution, and grown at 37°C in a 5% CO₂ atmosphere. The next day the medium was changed to DMEM containing the same additives except without fetal bovine serum, after which HPLC-purified APF or an equivalent amount of mock APF was added to each well. Live cell counts were performed on Day 3 of incubation by trypan blue exclusion. Values are the percent decrease in cell count compared to cell control given medium alone, and are given as the mean of triplicate wells; vertical lines are the standard deviation. The cells were sensitive to the antiproliferative activity of native purified APF (FIG. 9).

The APF composition may be delivered to the individual by any suitable means. In specific embodiments of the present invention, the APF composition is comprised as an oral medication and/or is delivered *via* a catheter, orally, intravenously, topically, subcutaneously, transcutaneously, intramuscularly, intra-jointly, parenterally, peritoneally, intranasally, intravesically, or by inhalation. A sufficient amount may be delivered directly to bladder tissue or it may be delivered systemically. A sufficient amount is one that ameliorates when given alone or in combination with other agents or other types of therapy at least one symptom or objective finding of the bladder cancer, and a skilled artisan recognizes standard methods to determine such an amount.

### PATENTS

U.S. Patent 5,811,393
U.S. Patent 5,916,871
U.S. Patent 5,962,645
U.S. Patent 6,156,522
U.S. Patent 6,232,289
U.S. Patent 6,376,197
U.S. Patent 6,600,018

### PUBLICATIONS

Ahn JM, Boyle NA, MacDonald MT, Janda KD Peptidomimetics and peptide backbone modifications. (2002) Mini Rev. Med. Chem., 2: 463-73
Aronov, O., Horowitz, A.T., Gabizon, A., Gibson, D. (2003) Bioconjug Chem 14(3):563-74.
Auger, G., Blanot, D., van Heijenoort, J., Nadal, C., Gournay, M. F., Winchenne, J. J., Boffa, G. A., Lambin, P., Maes, P., & Tartar, A. (1989) J Cell Biochem 40, 439-451.
Bafico, A., Gazit, A., Pramila, T., Finch, P. W., Yaniv, A., & Aaronson, S. A. (1999) J Biol Chem 274, 16180-16187.
Brensinger, C., Matthews, Y.L., Abele, S.T., Kusek, J.W., et al. (2001) Urology 57, 67-81.
Clugston PA, Vistnes MD, Perry LC, Maxwell GP, Fisher J (1995) Ann Plast Surg. Jan;34(1):12-5.
Curhan, G.C., Speizer, F.E., Hunter, D.J., Curhan, S.G., & Stampfer, M.J. (1999) J Urol 161, 549-552.
Di Stefano, G., Tubaro, M., Lanza, M., Boga, C., Fiume, L., Traldi, P. Rapid Commun Mass Spectrom 17(22):2503-7.
Heath, T.D. & Martin, F.J. (1986) Chem Phys Lipids 40(2-4):347-358.
Johansson, S. L. & Fall, M. (1990) J Urol 143, 1118-1124.
Jones, S.E., Jomarcy, C., Grist, J., Stewart, H.J., & Neal, M.J. (2000) Neuroreport 11, 3963-3967.
Jones, S.E., Jomacy, C. (2002) BioEssays 24, 811-820.
Keay, S., Zhang C-O, Shoenfelt J, Erickson DR, Whitmore K, Warren JW, Marvel R, & Chai T. (2001) Urology 57, 9-14.
Keay, S., Kleinberg, M., Zhang, C-O, Hise, M.K., & Warren, J.W. (2000) J Urol 64, 2112-2118.
Keay, S., Zhang, C-O., Shoenfelt, J. L., & Chai, T. C. (2003) Urology 61, 1278-1284.
Keay, S., Seillier-Moiseiwitsch, F., Zhang, C-O, Chai, T.C., & Zhang, J. (2003) Physiol Genomics 14, 107-115.
Keay, S., Zhang, C-O., Hise, M., Trifillis, A. L., Hebel, J. R., Jacobs, S. C., & Warren JW. (1996) J Urol 156, 2073-2078.
Keay, S., Zhang, C-O., Hise, M. K., Hebel, J. R., Jacobs, S. C., Gordon, D., Whitmore, K., Bodison, S., Gordon, N., & Warren, J. W. (1998) Urology 52, 974-978.
Leuck, M., & Kunz, H. (1997) J PraktChemie/Chemiker Zeitung 339, 322-334.
Mandler, R., Kobayashi, H., Hinson, E.R., Brechbiel, M.W., Waldmann, T.A. (2004) Cancer Res 64(4):1460-1467.
Moos, P. J., Fattaey, H. K., & Johnson, T. C. (1995) J Cell Biochem 59, 79-90.
Ou, X.H., Kuang, A.R., Peng, X., Zhong, Y.G. (2003) World J Gastroenterol 9(8):1675-8.
Pandur, P., Maurus, D., Kuhl, M. (2002) Bioessays 24, 881-884.
Polo M, Smith PD, Kim YJ, Wang X, Ko F, Robson MC (1999) Ann Plast Surg. Aug;43(2):185-90.
Qiu, D., Gandhi, S. S., & Koganty, R. R. (1996) Tetrahedron Letters 37, 595-598.
Safavy, A., Bonner, J.A., Waksal, H.W., Buchsbaum, D.J., Gillespie, G.Y., Khazaeli, M.B., Arani, R., Chen, D.T., Carpenter, M., Raisch, K.P. (2003) Bioconjug Chem 14(2):302-10.
Rashid, H.H., Reeder, J.E., O'Connell, M.J., Zhang, C.-O., Messing, E.M., and Keay, S.K. (2004) BMC Urology 4:3, 1-5.
Saitoh, T., Hirai, M., & Katoh, M. (2001) Int J Oncol 18, 991-996.
Schon, M.P. (1999) J Invest Dermatol. Sep;113(3):427.
Schumann, H., Holtz, J., Zerkowski, H.R., & Hatzfield, M. (2000) Cardiovasc Res 45, 720-728.
Sharifi, B.G. & Johnson, T.C. (1987) J Biol Chem 262, 15752-15755
Skoluda, D., Wegner, K., & Lemmel, E-M. (1974) Urologe 13, 15-23.
Tomaszewski, J.E., Landis, J.R., Russack, V., Williams, T.M., Wang, L.P., Hardy, C.,Svarovsky, S. A. &Barchi, J. J., Jr. (2003) Carbohydr Res 338, 1925-1935.
Thievessen, J., Seifert, H. H., Swiatkowski, S., Florl, A. R., & Schulz, W. A. (2003) Br J Cancer 88, 1932-1938.

## Claims

1. A synthetic glycopeptide which consists of:
(a) one to six sugar moieties; and
(b) a peptide whose sequence consists of the amino acid sequence of SEQ ID NO: 1, 3, 4, 5 or 6.

2. The glycopeptide of claim 1, further defined as a urinary bladder epithelial cell antiproliferative factor.

3. The glycopeptide of claim 1 or 2, wherein said sugar moiety comprises a sialic acid, galactose, glucose, N-acetylglucosamine and/or N-acetylgalactosamine.

4. The glycopeptide of any one of claims 1 to 3, wherein the linkage between a sugar and a peptide is in the alpha or beta configuration.

5. The glycopeptide of any one of claims 1 to 4, further defined as comprising:
(a) sialic acid-galactose-N-acetylgalactosamine-threonine-valine-proline-alanine-alanine-valine-valine-valine-alanine;
(b) sialic acid-galactose-N-acetylglucosamine-threonine-valine-proline-alanine-alanine-valine-valine-valine-alanine;
(c) sialic acid-galactose-N-acetylglucosamine-serine-leucine-proline-alanine-alanine-valine-valine-valine-alanine;
(d) galactose-N-acetylgalactosamine-threonine-valine-proline-alanine-alanine-valine-valine-valine-alanine;
(e) galactose-N-acetylglucosamine-threonine-valine-proline-alanine-alanine-valine-valine-valine-alanine; or
(f) galactose-N-acetylglucosamine-serine-leucine-proline-alanine-alanine-valine-valine-valine-alanine.

6. The glycopeptide of (a) in claim 5, further defined as having one or more of the following:
- the sialic acid is linked to galactose via a 2,3 linkage;
- the galactose is linked to the N-acetylgalactosamine via a 1,3 linkage; or
- the N-acetylgalactosamine is linked to threonine via an O linkage in an alpha configuration.

7. The glycopeptide of (b) or (c) in claim 5, further defined as having one or more of the following:
- the sialic acid is linked to galactose via a 2,3 linkage;
- the galactose is linked to the N-acetylglucosamine via a 1,4 linkage; or
- the N-acetylglucosamine is linked to threonine via an O linkage in an alpha configuration.

8. A composition comprising the glycopeptide of any one of claims 1 to 7 and a delivery vehicle.

9. The composition of claim 8, wherein the delivery vehicle is a liposome; a cell; a conjugated molecule optionally comprising an antibody, a peptide, folate, polyethylene glycol, a drug, a liposome, or lactosaminated human albumin; a nanoparticle optionally comprising colloidal gold; or a mixture thereof.

10. The glycopeptide of any one of claims 1 to 7 or the composition of claim 8 or 9 comprised in a pharmaceutically acceptable excipient.

11. The glycopeptide or composition of any one of claims 1 to 10, which reversibly arrests cell proliferation.

12. The glycopeptide or composition of any one of claims 1 to 11, which comprises activity for arresting cell cycling in G2.

13. A kit, comprising the glycopeptide or composition of any one of claims 1 to 12 housed in a suitable container.

14. The glycopeptide or composition of any one of claims 1 to 12 for use in a method of treating cancer or a hyperplasia.

15. The glycopeptide or composition for use according to claim 14, wherein the cancer comprises an epithelial cancer; bladder cancer; or prostate cancer.

## Patentansprüche

1. Synthetisches Glycopeptid, das aus
(a) einer bis sechs Zuckergruppierungen und
(b) einem Peptid, dessen Sequenz aus der Aminosäuresequenz SEQ ID NO: 1, 3, 4, 5 oder 6 besteht, besteht.

2. Glycopeptid nach Anspruch 1, das außerdem als antiproliferativer Faktor von Harnblasenepithelzellen definiert ist.

3. Glycopeptid nach Anspruch 1 oder 2, wobei die Zuckergruppierung Sialinsäure, Galactose, Glucose, N-Acetylglucosamin und/oder N-Acetylgalactosamin umfasst.

4. Glycopeptid nach einem der Ansprüche 1 bis 3, wobei die Verknüpfung zwischen einem Zucker und einem Peptid in der alpha- oder beta-Konfiguration ist.

5. Glycopeptid nach einem der Ansprüche 1 bis 4, das außerdem definiert ist als umfassend:
(a) Sialinsäure-galactose-N-acetylgalactosamin-threonin-valin-prolin-alanin-alanin-valin-valin-valin-alanin;
(b) Sialinsäure-galactose-N-acetylglucosamin-threonin-valin-prolin-alanin-alanin-valin-valin-valin-alanin;
(c) Sialinsäure-galactose-N-acetylglucosamin-serin-leucin-prolin-alanin-alanin-valin-valin-valin-alanin;
(d) Galactose-N-acctylgalactosamin-threonin-valin-prolin-alanin-alanin-valin-valin-valin-alanin;
(e) Galactose-N-acetylglucosamin-threonin-valin-prolin-alanin-alanin-valin-valin-valin-alanin oder
(f) Galactose-N-acetylglucosamin-serin-leucin-prolin-alanin-alanin-valin-valin-valin-alanin.

6. Glycopeptid von (a) in Anspruch 5, das außerdem als eines oder mehrere der Folgenden aufweisend definiert ist:
- die Sialinsäure ist über eine 2,3-Verknüpfung mit Galactose verknüpft;
- die Galactose ist über eine 1,3-Verknüppfung mit dem N-Acetylgalactosamin verknüpft oder
- das N-Acetylgalactosamin ist über eine O-Verknüpfung in einer alpha-Konfiguration mit Threonin verknüpft.

7. Glycopeptid von (b) oder (c) in Anspruch 5, das außerdem als eines oder mehrere der Folgenden aufweisend definiert ist:
- die Sialinsäure ist über eine 2,3-Verknüpfung mit Galactose verknüpft;
- die Galactose ist über eine 1,4-Verknüpfung mit dem N-Acetylglucosamin verknüpft oder
- das N-Acetylglucosamin ist über eine O-Verknüpfung in einer alpha-Konfiguration mit Threonin verknüpft.

8. Zusammensetzung, die das Glycopeptid nach einem der Ansprüche 1 bis 7 und ein Abgabevehikel umfasst.

9. Zusammensetzung nach Anspruch 8, wobei das Abgabevehikel ein Liposom; eine Zelle; ein konjugiertes Molekül, das gegebenenfalls einen Antikörper, ein Peptid, Folat, Polyethylenglycol, ein Arzneimittel, ein Liposom oder lactosaminiertes humanes Albumin umfasst; ein Nanopartikel, das gegebenenfalls kolloidales Gold umfasst; oder ein Gemisch davon ist.

10. Glycopeptid nach einem der Ansprüche 1 bis 7 oder Zusammensetzung nach Anspruch 8 oder 9, das/die in einem pharmazeutisch annehmbaren Exzipiens enthalten ist.

11. Glycopeptid oder Zusammensetzung nach einem der Ansprüche 1 bis 10, das/die die Zellproliferation reversibel anhält.

12. Glycopeptid oder Zusammensetzung nach einem der Ansprüche 1 bis 11, das/die Aktivität zum Anhalten des Zellzyklus in G2 umfasst.

13. Kit, umfassend das Glycopeptid oder die Zusammensetzung nach einem der Ansprüche 1 bis 12, untergebracht in einem geeigneten Behälter.

14. Glycopeptid oder Zusammensetzung nach einem der Ansprüche 1 bis 12 zur Verwendung in einem Verfahren zur Behandlung von Krebs oder Hyperplasie.

15. Glycopeptid oder Zusammensetzung zur Verwendung nach Anspruch 14, wobei der Krebs einen Epithelkrebs; Blasenkrebs oder Prostatakrebs umfasst.

## Revendications

1. Glycopeptide synthétique, constitué :
a) de un à six motifs de sucre,
b) et d'un peptide dont la séquence consiste en la séquence d'acides aminés présentée en tant que Séquence N° 1, 3, 4, 5 ou 6.

2. Glycopeptide conforme à la revendication 1, défini en outre comme étant un facteur antiprolifératif spécifique des cellules épithéliales de la vessie urinaire.

3. Glycopeptide conforme à la revendication 1 ou 2, dans lequel ledit motif de sucre comprend un acide sialique, galactose, glucose, N-acétyl-glucosamine et/ou N-acétyl-galactoamine

4. Glycopeptide conforme à l'une des revendications 1 à 3, dans lequel la liaison entre un sucre et un peptide se présente en configuration alpha ou bêta.

5. Glycopeptide conforme à l'une des revendications 1 à 4, plus précisément défini comme comprenant :
a) acide sialique - galactose - N-acétyl-galactosamine - thréonine - valine - proline - alanine - alanine - valine - valine - valine - alanine ;
b) acide sialique - galactose - N-acétyl-glucosamine - thréonine - valine - proline - alanine - alanine - valine - valine - valine - alanine ;
c) acide sialique - galactose - N-acétyl-glucosamine - sérine - leucine - proline - alanine - alanine - valine - valine - valine - alanine ;
d) galactose - N-acétyl-galactosamine - thréonine - valine - proline - alanine - alanine - valine - valine - valine - alanine ;
e) galactose - N-acétyl-glucosamine - thréonine - valine - proline - alanine - alanine - valine - valine - valine - alanine ;
f) ou galactose - N-acétyl-glucosamine - sérine - leucine - proline - alanine - alanine - valine - valine - valine - alanine.

6. Glycopeptide conforme à la revendication 5, item a), plus précisément défini comme présentant l'une ou plusieurs des particularités suivantes :
- l'acide sialique est rattaché au galactose par une liaison 2-3 ;
- le galactose est rattaché à la N-acétyl-galactosamine par une liaison 1-3 ;
- la N-acétyl-galactosamine est rattachée à la thréonine par une liaison O-glycosidique en configuration alpha.

7. Glycopeptide conforme à la revendication 5, item b) ou c), plus précisément défini comme présentant l'une ou plusieurs des particularités suivantes :
- l'acide sialique est rattaché au galactose par une liaison 2-3 ;
- le galactose est rattaché à la N-acétyl-glucosamine par une liaison 1-4 ;
- la N-acétyl-glucosamine est rattachée à la thréonine par une liaison O-glycosidique en configuration alpha.

8. Composition comprenant un glycopeptide, conforme à l'une des revendications 1 à 7, et un véhicule d'administration.

9. Composition conforme à la revendication 8, dans laquelle le véhicule d'administration est
- un liposome ;
- une cellule ;
- une molécule conjuguée, comprenant, en options, un anticorps, un peptide, du folate, du polyéthylèneglycol, un médicament, un liposome, ou de l'albumine humaine lactosaminée ;
- une nanoparticule, comprenant, en option, de l'or colloïdal ;
- ou un mélange de tels véhicules.

10. Glycopeptide conforme à l'une des revendications 1 à 7, ou composition conforme à la revendication 8 ou 9, compris(e) dans un excipient pharmacologiquement admissible.

11. Glycopeptide ou composition conforme à l'une des revendications 1 à 10, qui arrête réversiblement la prolifération cellulaire.

12. Glycopeptide ou composition conforme à l'une des revendications 1 à 11, qui possède une activité consistant à arrêter le cycle cellulaire en phase G2.

13. Trousse comprenant un glycopeptide ou une composition conforme à l'une des revendications 1 à 12, logé(e) dans un récipient approprié.

14. Glycopeptide ou composition conforme à l'une des revendications 1 à 12, pour utilisation dans un procédé de traitement d'un cancer ou d'une hyperplasie.

15. Glycopeptide ou composition pour utilisation conforme à la revendication 14, le cancer comprenant un cancer épithélial, un cancer de la vessie ou un cancer de la prostate.
